# EUROPEAN PATENT APPLICATION

(11) **EP 4 303 302 A1**
(43) Date of publication of application: **10.01.2024**
(21) Application number: 22763287.4
(22) Date of filing: 02.03.2022
(51) Int. Cl.: C12N 15/09, A01H 5/00, A01K 67/027, C12M 1/34, C12N 1/15, C12N 1/19, C12N 5/10, C12Q 1/6869

(54) **GENOMIC DNA MODIFICATION METHOD AND MODIFICATION DETECTION METHOD**

(30) Priority: 03.03.2021 JP 2021033658
(71) Applicant: Logomix, Inc., Tokyo 104-0053 (JP)
(72) Inventor: AIZAWA, Yasunori, Yokohama-shi, Kanagawa 246-0012 (JP)
(74) Representative: Hoffmann Eitle
(86) International application number: PCT/JP2022/008713
(87) International publication number: WO 2022/186233

(57) **Abstract**

The present invention provides a method of *in vitro* modification of genomic DNA in a eukaryotic cell and a method of detecting modification. The present invention can involve, for example, introducing a nucleotide sequence having 50% or more sequence identity to any one of nucleotide sequences of retrotransposons of genomic DNA. The present invention can also involve changing an insertion position, to each neighboring sequence, of at least one of nucleotide sequences of retrotransposons. The present invention provides a modified eukaryotic cell obtained by such a method.

## Description

### Technical Field

The present invention relates to a method of modify ing a genomic DNA and a method of detecting the modification. The present invention relates to, for example, a method comprising introducing a nucleotide sequence having 50% or more sequence identity to any one of nucleotide sequence of retrotransposons of genomic DNA. The present invention also relates to a method comprising changing an insertion position, to each neighboring sequence, of at least one of nucleotide sequences of retrotransposons. The present invention relates to a modified eukaryotic cell obtained by such a method.

### Background Art

Techniques of labeling the genome of cells are useful as techniques of tracking the origin of cells and have been developed. For example, Patent Literatures 1 to 3 disclose a method for introducing information, a coding sequence, or a code to the genomic DNA of cells. Patent Literatures 1 to 3 disclose that retrotransposons are used for introducing information or a coding sequence.

Techniques using the nucleotide sequences of retrotransposons have been developed as methods for variety identification, quality management, and distribution management of agricultural products. For example, Non Patent Literature 1 discloses a method for identifying a sweet potato variety based on insertion polymorphisms of the nucleotide sequences of endogenous retrotransposons by analyzing the genomic DNA of sweet potato.

### Citation List

### Patent Literature

Patent Literature 1: US2010/0281555
Patent Literature 2: WO2020/247685A
Patent Literature 3: WO2000/068431A

### Non Patent Literature

Non Patent Literature 1: Monden Y. et al., DNA Research, 21 (5): 491-498, 2014

### Summary of Invention

The present invention provides a method of modifying genomic DNA in a eukaryotic cell and a method of detecting the modification. The present invention can involve, for example, introducing a nucleotide sequence having 50% or more sequence identity to any one of nucleotide sequence of retrotransposons of genomic DNA. The present invention can also involve changing an insertion position of at least one of nucleotide sequences of retrotransposons to each neighboring sequence. The present invention can further involve modifying at least one of nucleotide sequences of retrotransposons. The present invention provides a modified eukaryotic cell obtained by such a method.

Eukaryotic cells contain the sequences of many retrotransposons, and the retrotransposons are capable of causing a polymorphism (i.e., an insertion polymorphism) that differs in insertion position among cells or individuals of the same organism species. Due to this insertion polymorphism, the modification of cells can be identified in the cells having the modification on the basis of modification information thereon, whereas the modification can be used as impossible-to-tamper labeling for cells through the use of the fact that the modification cannot be identified without the basis of the modification information.

The present invention provides the following aspects.
(1) A method for obtaining a cell, wherein
   the cell comprises genomic DNA and is preferably a cloned cell, and the genomic DNA comprises nucleotide sequences of a plurality of retrotransposons (preferably transpositionally active retrotransposon),
   the method comprising:
      (i) newly introducing a nucleotide sequence having 50% or more sequence identity to any one of the nucleotide sequences of the retrotransposons in the genomic DNA (i.e., a retrotransposon or retrotransposon-like nucleotide sequence) into a target sequence in the genomic DNA, and selecting a cell comprising the genomic DNA with the retrotransposon or retrotransposon-like sequence integrated in the target sequence;
      (ii) changing an insertion position, to each neighboring sequence, of at least one of the nucleotide sequences of the retrotransposons in the genomic DNA, and selecting a cell comprising the genomic DNA having the nucleotide sequence of the retrotransposon with the insertion position changed; and/or
      (iii) modifying (nucleotide sequence modification selected from insertion, substitution, deletion, addition, and elimination) nucleotide sequences of one or more transposons of the genomic DNA, and selecting a cell in which the nucleotide sequence of the transposon at a particular position of the genomic DNA is a sequence different from the natural one.
(2) The method according to (1), wherein in the (i), the new introduction into the target sequence comprises introducing the retrotransposon or retrotransposon-like sequence into the target sequence of the genomic DNA through the use of nucleotide sequence-specific cleavage and subsequent homologous recombination, and selecting a cell comprising the genomic DNA with the retrotransposon or retrotransposon-like sequence integrated in the target sequence.
(3) The method according to (2), wherein in the (i), the new introduction into the target sequence further comprises, upon the introduction, removing a portion or the whole of an artificial sequence integrated in the genomic DNA from the genomic DNA, and selecting a cell from which a portion or the whole of the integrated artificial sequence has been removed.
(4) The method according to any of (1) to (3), wherein the nucleotide sequence to be newly introduced in the (i) has 90% or more sequence identity to any one of the nucleotide sequences of the retrotransposons in the genomic DNA.
(5) The method according to any of (1) to (4), wherein the nucleotide sequence to be newly introduced in the (i) is a nucleotide sequence identical to any one of the nucleotide sequences of the retrotransposons in the genomic DNA.
(6) The method according to any of (1) to (5), further comprising (i) introducing a nucleotide sequence having 50% or more sequence identity to any one of the nucleotide sequences of the retrotransposons in the genomic DNA (second nucleotide sequence) into another target sequence (second target sequence) in the genomic DNA.
(7) A cell having, in a target sequence in genomic DNA, an additional nucleotide sequence having 50% or more sequence identity to any one of nucleotide sequences of retrotransposons in the genomic DNA.
   (7A) The method according to any of (1) to (6), further comprising (ii) changing insertion positions, to each neighboring sequence, of at least two of the nucleotide sequences of the retrotransposons.
(8) The cell according to (7), wherein the additional nucleotide sequence is a non-natural nucleotide sequence. (8A) (8a) A cell having genomic DNA having a nucleotide sequence of a retrotransposon with an insertion position to a neighboring sequence changed, or
   (8b) a cell having genomic DNA having modification in a portion of a nucleotide sequence of a retrotransposon.
(9) A method for analyzing the origin of a cell or genomic DNA thereof, or a modified region-containing fragment of the genomic DNA, the method comprising:
   decoding a nucleotide sequence of a modified region in the genomic DNA of the test cell suspected of being derived from the cell according to (7) or (8) (cell of origin), the test genomic DNA, or the modified region-containing fragment of the genomic DNA, or providing information on the decoded nucleotide sequence;
   determining or estimating the presence or absence of a nucleotide sequence introduced in the modified region on the basis of the decoded nucleotide sequence and information on the modified region; and
   when the sequence introduced in the modified region is determined or estimated to be present, determining or estimating that the test cell is derived or possibly derived from the cell according to (7) or (8) (the cell of origin), and/or in other cases, determining or estimating that the test cell is not derived or possibly not derived from the cell according to (7) or (8) (the cell of origin).
(10) A non-human organism comprising the cell according to (7) or (8).
(11) A cell management system comprising a plurality of containers each containing the cell according to (7) or (8), wherein the containers have readable information corresponding to each of the containers, and further have a correspondence table comprising a modified region of each cell associated with the readable information.
(12) A method comprising:
   providing readable information corresponding to each of a plurality of containers each containing the cell according to (7) or (8) or genomic DNA thereof, or a modified region-containing fragment of the genomic DNA, and a correspondence table comprising information on a modified region of each cell associated with the readable information; and
   identifying information on a modified region in the cell contained in the container, the genomic DNA thereof, or the modified region-containing fragment of the genomic DNA from the readable information given to the container on the basis of the correspondence table, and/or identifying a cell having the modification or genomic DNA thereof, or a modified region-containing fragment of the genomic DNA, or a container containing it from the information on the modified region.
(13) The method according to (12), further comprising:
   providing a plurality of containers each containing the cell according to (7) or (8) or genomic DNA thereof, or a modified region-containing fragment of the genomic DNA, the containers being each given readable information; and
   storing or preserving the containers under conditions suitable for the storage or preservation of the cell, the genomic DNA thereof, or the modified region-containing fragment of the genomic DNA.
(14) A method comprising:
   providing information on each of animal or plant (e.g., livestock) individuals or varieties each comprising the cell according to (7) or (8), and a correspondence table comprising modification information on genomic DNA of each animal or plant (e.g., livestock) individual or variety associated with the information; and
   identifying modification information on the genomic DNA from the information on the animal or plant (e.g., livestock) individual or variety on the basis of the correspondence table, and/or identifying an animal or plant (e.g., livestock) individual or variety having the modification from the modification information on the genomic DNA.
(15) A management system of information on a cell or genomic DNA thereof, or a modified region-containing fragment of the genomic DNA, the management system comprising:
   a recording medium that stores readable information corresponding to each of a plurality of containers each containing the cell according to (7) or (8) or genomic DNA thereof, or a modified region-containing fragment of the genomic DNA, and a correspondence table comprising modification information on each cell associated with the readable information;
   a reception part that receives the readable information and/or the modification information; and
   a processor capable of executing a program, wherein the program is a program to identify modification information on the cell contained in the container having the received readable information, the genomic DNA thereof, or the modified region-containing fragment of the genomic DNA on the basis of the correspondence table read from the recording medium, and/or to identify a cell or genomic DNA thereof, or a modified region-containing fragment of the genomic DNA, or a container containing it from the modification information.
(16) An animal or plant (e.g., livestock) individual or variety management system comprising:
   a recording medium that stores information on each of a plurality of animal or plant (e.g., livestock) individuals or varieties each comprising the cell according to (7) or (8), and a correspondence table comprising modification information on genomic DNA of each animal or plant (e.g., livestock) individual or variety associated with the information;
   a reception part that receives the information on each of the plurality of animal or plant (e.g., livestock) individuals or varieties, and/or the modification information on the genomic DNA; and/or
   a processor capable of executing a program, wherein the program is a program to identify modification information on the genomic DNA of an animal or plant (e.g., livestock) individual or variety corresponding to the received information on each of the animal or plant (e.g., livestock) individuals or varieties on the basis of the correspondence table read from the recording medium, and/or to identify an animal or plant (e.g., livestock) individual or variety having modification corresponding to the modification information on the genomic DNA.
(17) The cell management system according to (15), further comprising a storeroom (e.g., a storeroom containing liquid nitrogen, or a freezer, for example, a freezer of -80°C to -20°C) comprising a plurality of containers each containing the cell according to (7) or (8) or genomic DNA thereof, or a modified region-containing fragment of the genomic DNA.
(18) The method according to any of (1) to (6), the cell according to (7) or (8), the method according to (9), or the non-human organism according to (10), wherein the retrotransposons are transpositionally active retrotransposons.
(19) The method according to any of (1) to (6), wherein the method comprises (i).
(20) The method according to any of (1) to (6), wherein the method comprises (ii).
(21) The method according to any of (1) to (6), wherein the method comprises (iii).

### Brief Description of Drawings

[Figure 1] Figure 1 shows one example of a method for introducing a retrotransposon or retrotransposon-like sequence to genomic DNA.
[Figure 2] Figure 2 shows one example of a method for introducing a retrotransposon or retrotransposon-like sequence to genomic DNA.
[Figure 3] Figure 3 shows one example of a method for introducing a retrotransposon or retrotransposon-like sequence to genomic DNA.
[Figure 4] Figure 4 shows one example of a method for introducing a retrotransposon or retrotransposon-like sequence to genomic DNA.

### Description of Embodiments

In the present specification, the term "eukaryote" is an organism having cells having a cell nucleus. The eukaryote is not particularly limited and can be an animal or a plant. Examples of the animal include agricultural animals such as mammals (non-human mammals), particularly, livestock, for example, bovines, pigs, goats, sheep, horses, llamas, and camels, birds such as chickens, and marine products such as fish, pet animals such as dogs, cats, rabbits, guinea pigs, hamsters, and mice, and insects. Examples of the plants include agricultural crops, for example, edible crops, for example, rice, corn, tubers and roots, beans, grains such as barley and wheat, horticultural crops (vegetables, fruit trees, and flowers), for example, leaf vegetables (cabbage, asparagus, etc.), fruit vegetables (eggplant, tomato, cucumbers, etc.), root vegetables (radish, carrot, etc.), other vegetables, fruits (e.g., pomaceous fruits such as apple and pear, stone fruits such as Japanese apricot, apricot, prune, peach, and cherry, nuts such as almond, walnut, and Japanese chestnut, citruses such as tangerine and lemon, and tropical fruit trees such as tropical fruits), and ornamental plants, and seeds, seedlings, and bulbs of any of these plants. The eukaryote can also be a microalga. The eukaryote can be a human or a non-human animal.

In the present specification, the term "cell" refers to a fundamental unit of life having at least genomic DNA, cytoplasm, and a membrane structure that surrounds these components. Examples of the cell include, but are not particularly limited to, cells of prokaryotes and cells of eukaryotes. The genomic DNA comprises endogenous DNA of the cell and however, is not necessarily composed of only endogenous factors of the cell. In the present specification, a cell of a eukaryote or a eukaryotic cell is a cell of a eukaryote. The eukaryotic cell may be a human cell. The cell can be a purified cell or an isolated cell. The cell may be a cultured cell. The cell may be an established cell line. The cell can be, for example, a somatic cell. The cell can be, for example, a pluripotent cell or a pluripotent stem cell. The cell can be, for example, a tissue stem cell. The cell can be, for example, a tissue progenitor cell. The cell can be, for example, a germ cell.

The cell contains the genomic DNA of the cell and may further contain genomic DNA of a foreign invader (e.g., a pathogen). In the present specification, the genomic DNA refers to the genomic DNA of the cell itself (i.e., host genomic DNA) unless otherwise specified. The genomic DNA of an invader is capable of residing in the cell independently of the host genomic DNA and may be integrated into the host genomic DNA. The host genomic DNA can involve a foreign factor (e.g., an insert of the whole or a portion of genomic DNA of a virus or the like).

In the present specification, the term "cell population" means a composition including a plurality of cells.

In the present specification, the term "isolation" means the separation of cells of interest from at least one of other components. The isolation can be carried out, for example, by separating and taking cells in a natural state of existence from other components existing together therewith in a natural state of existence. The isolation can be carried out, for example, by separating and taking some cells from a multicellular organism. In the present specification, a technique of handling isolated cells is referred to as an *in vitro* technique.

In the present specification, the term "purification" means the further separation of isolated cells of interest from other components existing together therewith. The purification can be caried out, for example, by separating the cells of interest from other components on the basis of morphology or a surface marker. The purification can be carried out by limiting dilution and/or cloning of cells. The purification can be carried out by establishing a cell line from the cells of interest. When the cells of interest have a marker gene such as a drug resistance gene or a gene encoding a fluorescent protein, the purification can be carried out on the basis of the expression of the marker gene. In the present specification, the term "enrich" means improvement in the existence density of the cells of interest.

In the present specification, the terms "polynucleotide" and "nucleic acid" are used interchangeably with each other and each refer to a nucleotide polymer in which nucleotides are linked through phosphodiester bonds. The "polynucleotide" or the "nucleic acid" may be DNA, may be RNA, or may be constituted by a combination of DNA and RNA. The "polynucleotide" or the "nucleic acid" may be a polymer of natural nucleotides, may be a polymer of natural nucleotides and non-natural nucleotides (analogs of natural nucleotides, nucleotides modified at one of their base moiety, sugar moiety and phosphate moiety (e.g., phosphorothioate skeletons), etc.), or may be a polymer of non-natural nucleotides.

In the present specification, the nucleotide sequence of the "polynucleotide" or the "nucleic acid" is described by generally accepted single-letter codes unless otherwise specified. Each nucleotide sequence is described from the 5' side toward the 3' side unless otherwise specified. The nucleotide residues constituting the "polynucleotide" or the "nucleic acid" may be simply described by adenine, thymine, cytosine, guanine, or uracil, etc., or their single-letter codes.

In the present specification, the term "gene" refers to a polynucleotide containing at least one open reading frame encoding a particular protein. The gene can contain both an exon and an intron.

In the present specification, the terms "polypeptide", "peptide" and "protein" are used interchangeably with each other and each refer to a polymer of amino acids linked through amide bonds. The "polypeptide", the "peptide" or the "protein" may be a polymer of natural amino acids, may be a polymer of natural amino acids and non-natural amino acids (chemical analogs, modified derivatives, etc. of natural amino acids), or may be a polymer of non-natural amino acids. Each amino acid sequence is described from the N-terminal side toward the C-terminal side unless otherwise specified.

In the present specification, the term "alleles" refer to a set of nucleotide sequences present at the same locus on the chromosomal genome. In an aspect, a diploid cell has two alleles at the same locus, and a triploid cell has three alleles at the same locus. In an aspect, an additional allele may be formed by an abnormal copy of the chromosome or an abnormal additional copy of the locus.

In the present specification, the terms "genome modification" and "genome editing" are used interchangeably with each other and each refer to introduction of a mutation at a desired position (target region) in the genome. The genome modification can involve using (particularly, introduction to a cell) a sequence-specific nucleic acid cleaving molecule (e.g., sequence-specific or sequence-dependent endonuclease) designed so as to cleave DNA of the target region. In a preferred embodiment, the genome modification can involve using nuclease engineered so as to cleave DNA of the target region. In a preferred embodiment, the genome modification can involve using nuclease (e.g., TALEN or zinc finger nuclease (ZFN)) engineered so as to cleave a target sequence having a particular nucleotide sequence in the target region. In a particularly preferred embodiment, the genome modification can involve using nuclease (e.g., CRISPR-Cas9 system) engineered so as to cleave a target sequence having a particular nucleotide sequence in the target region. In a preferred embodiment, the genome modification may employ sequence-specific endonuclease such as a restriction enzyme (e.g., meganuclease) having only one cleavage site in the genome (e.g., a restriction enzyme having 16-base sequence specificity (theoretically, which is present at a ratio of 1 out of 4¹⁶ bases), a restriction enzyme having 17-base sequence specificity (theoretically, which is present at a ratio of 1 out of 4¹⁷ bases), and a restriction enzyme having 18-base sequence specificity (theoretically, which is present at a ratio of 1 out of 4¹⁸ bases)) so as to cleave a target sequence having a particular nucleotide sequence in the target region. Typically, use of site-specific nuclease induces double-strand break (DSB) in DNA of the target region, followed by the repair of the genome by an endogenous process of cells, such as homologous directed repair (HDR) and non-homologous end-joining repair (NHEJ). NHEJ is a repair method of linking ends that have undergone double-strand break, without the use of a donor DNA, and induces insertion and/or deletion (indel) with high frequency during the repair. HDR is a repair mechanism using a donor DNA and is also capable of introducing a desired mutation to a target region. Examples of the genome modification technique preferably include a CRISPR/Cas system. The meganuclease that can be used is, for example, meganuclease selected from the group consisting of I-SceI, I-SceII, I-SceIII, I-SceIV, I-SceV, I-SceVI, I-SceVII, I-CeuI, I-CeuAIIP, I-CreI, I-CrepsbIP, I-CrepsbIIP, I-CrepsbIIIP, I-CrepsbIVP, I-TliI, I-PpoI, PI-PspI, F-SceI, F-SceII, F-SuvI, F-TevI, F-TevII, I-AmaI, I-AniI, I-ChuI, I-CmoeI, I-CpaI, I-CpaII, I-CsmI, I-CvuI, I-CvuAIP, I-DdiI, I-DdiII, I-DirI, I-DmoI, I-HmuI, I-HmuII, I-HsNIP, I-LlaI, I-MsoI, I-NaaI, I-NanI, I-NclIP, I-NgrIP, I-NitI, I-NjaI, I-Nsp236IP, I-PakI, I-PboIP, I-PcuIP, I-PcuAI, I-PcuVI, I-PgrIP, I-PobIP, I-PorI, I-PorIIP, I-PbpIP, I-SpBetaIP, I-ScaI, I-SexIP, I-SneIP, I-SpomI, I-SpomCP, I-SpomIP, I-SpomIIP, I-SquIP, I-Ssp68031, I-SthPhiJP, I-SthPhiST3P, I-SthPhiSTe3bP, I-TdeIP, I-TevI, I-TevII, I-TevIII, I-UarAP, I-UarHGPAIP, I-UarHGPA13P, I-VinIP, I-ZbiIP, PI-Mtul, PI-MtuHIP PI-MtuHIIP, PI-PfuI, PI-PfuII, PI-PkoI, PI-PkoII, PI-Rma43812IP, PI-SpBetaIP, PI-SceI, PI-TfuI, PI-TfuII, PI-ThyI, PI-TliI, and PI-TliII and their functional derivative restriction enzymes, or a cleavage site (or a recognition site) thereof, preferably meganuclease which is a restriction enzyme having 18-base or more sequence specificity, or a cleavage site (or a recognition site) thereof, particularly, meganuclease that does not cleave one location or two or more locations of the genome in a cell, or a cleavage site thereof.

The term "target region" refers to a region that is targeted by a genome modification system. In the present invention, a DNA region on the genome positioned between target regions at two locations (e.g., a first target region and a second target region) can be deleted.

The term "sequence-specific nucleic acid cleaving molecule" refers to a molecule that can recognize a particular nucleic acid sequence and cleave a nucleic acid at the particular nucleic acid sequence. The sequence-specific nucleic acid cleaving molecule is a molecule having activity of cleaving a nucleic acid in a sequence-specific manner (sequence-specific nucleic acid cleaving activity).

The term "target sequence" refers to a DNA sequence, in the genome, to be cleaved by the sequence-specific nucleic acid cleaving molecule. When the sequence-specific nucleic acid cleaving molecule is Cas protein, the target sequence refers to a DNA sequence, in the genome, to be cleaved by the Cas protein. In the case of using Cas9 protein as the Cas protein, the target sequence needs to be a sequence adjacent to the 5' side of a protospacer adjacent motif (PAM). The target sequence is usually selected as a sequence of 17 to 30 bases (preferably 18 to 25 bases, more preferably 19 to 22 bases, further preferably 20 bases) immediately adjacent to the 5' side of PAM. The target sequence can be designed using a design tool known in the art such as CRISPR DESIGN (crispr.mit.edu/).

The term "Cas protein" refers to CRISPR-associated protein. In a preferred aspect, the Cas protein forms a complex with guide RNA and exhibits endonuclease activity or nickase activity. Examples of the Cas protein include, but are not particularly limited to, Cas9 protein. The Cas protein encompasses wild-type Cas protein and its homologs (paralogs and orthologs), and their mutants as long as they exhibit endonuclease activity or nickase activity in cooperation with guide RNA.

In a preferred aspect, the Cas protein is involved in a class 2 CRISPR/Cas system and more preferably involved in a type II CRISPR/Cas system. Preferred examples of the Cas protein include Cas9 protein.

The term "Cas9 protein" refers to Cas protein that is involved in a type II CRISPR/Cas system. The Cas9 protein forms a complex with guide RNA and exhibits activity of cleaving DNA of a target region in cooperation with the guide RNA. The Cas9 protein encompasses wild-type Cas9 protein and its homologs (paralogs and orthologs), and their mutants as long as they exhibit the activity described above. The wild-type Cas9 protein has a RuvC domain and a HNH domain as nuclease domains. In the present specification, any one of the RuvC domain and the HNH domain in the Cas9 protein may be inactivated. Cas9 in which any one of the RuvC domain and the HNH domain is inactivated introduces single-strand cleavage (nick) in double-stranded DNA. Hence, in the case of using Cas9 in which any one of the RuvC domain and the HNH domain is inactivated in the cleavage of double-stranded DNA, a modification system can be configured such that a target sequence of Cas9 is set in each of the sense strand and the antisense strand and nick for the sense strand and nick for the antisense strand occur at sufficiently close positions, thereby inducing double-strand break.

Examples of the organism species from which the Cas9 protein is derived preferably include, but are not particularly limited to, bacteria belonging to the genus *Streptococcus,* the genus *Staphylococcus,* the genus *Neisseria,* or the genus *Treponema.* More specifically, examples thereof preferably include Cas9 protein derived from *S. pyogenes, S. thermophilus, S. aureus, N. meningitidis,* or *T. denticola.* In a preferred aspect, the Cas9 protein is *S. pyogenes*-derived Cas9 protein.

The terms "guide RNA" and "gRNA" are used interchangeably with each other and each refer to RNA that can form a complex with Cas protein and lead the Cas protein to a target region. In a preferred aspect, the guide RNA comprises CRISPR RNA (crRNA) and transactivating CRISPR RNA (tracrRNA). crRNA is involved in binding to a target region in the genome, and tracrRNA is involved in binding to the Cas protein. In a preferred aspect, crRNA comprises a spacer sequence and a repeat sequence, and the spacer sequence binds to a complementary strand of a target sequence in the target region. In a preferred aspect, tracrRNA comprises an anti-repeat sequence and a 3' tail sequence. The anti-repeat sequence has a sequence complementary to the repeat sequence of crRNA and forms base pairs with the repeat sequence. The 3' tail sequence usually forms three stem loops.

The guide RNA may be single-guide RNA (sgRNA) in which the 5' end of tracrRNA is linked to the 3' end of crRNA, or may be formed by the base pairing of the repeat sequence and the anti-repeat sequence of crRNA and tracrRNA prepared as separate RNA molecules. In a preferred aspect, the guide RNA is sgRNA.

The repeat sequence of crRNA and the sequence of tracrRNA can be appropriately selected according to the type of the Cas protein, and sequences derived from the same bacterial species as that for the Cas protein can be used. S. *pyogenes*-derived Cas9 protein, crRNA, and tracrRNA (or sgRNA) can be used as CRISPR-Cas9 system. Various crRNA repeat sequences and tracrRNA sequences for sgRNA design have been proposed. Those skilled in the art can design sgRNA on the basis of a technique known in the art (e.g., Jinek et al., (2012) Science, 337, 816-21; Mali et al., (2013) Science, 339: 6121, 823-6; Cong et al., (2013) Science, 339: 6121, 819-23; Hwang et al., (2013) Nat. Biotechnol. 31: 3, 227-9; and Jinek et al., (2013) eLife, 2, e00471).

The term "operably linked" used in relation to a polynucleotide means that a control sequence such as a promoter is placed sufficiently close to a gene sequence so that the control sequence such as a promoter is capable of influencing the expression of the gene sequence. For example, the phrase "polynucleotide is functionally linked to a promoter" means that the polynucleotide is linked so as to be expressed under the control of the promoter.

The term "expressible state" refers to a state in which a polynucleotide can be transcribed in a cell harboring the polynucleotide.

The term "expression vector" is a vector containing a subject polynucleotide and refers to a vector having a system that puts the subject polynucleotide in an expressible state in a cell harboring the vector. For example, the "Cas protein expression vector" means a vector that permits expression of the Cas protein in a cell harboring the vector. For example, the "guide RNA expression vector" means a vector that permits expression of the guide RNA in a cell harboring the vector.

In the present specification, the sequence identity (or homology) between nucleotide sequences or amino acid sequences is determined as the ratio of identical bases or amino acids to the whole nucleotide sequences or the whole amino acid sequences, except for gaps, in alignments obtained by juxtaposing two nucleotide sequences or amino acid sequences so as to attain the highest identity of the corresponding bases or amino acids while placing the gaps in moieties corresponding to insertion and deletion. The sequence identity between nucleotide sequences or amino acid sequences can be determined using various homology search software known in the art. For example, the value of sequence identity between nucleotide sequences can be obtained by calculation based on alignments obtained with homology search software BLASTN known in the art, and the value of sequence identity between amino acid sequences can be obtained by calculation based on alignments obtained with homology search software BLASTP known in the art.

In the present specification, the term "transposon" is a mobile genetic element present in genomic DNA. The transposon is universally present in the genomic DNA of eukaryotic cells. A retrotransposon is a transposon of type that replicates its own DNA into genomic DNA by transcribing its copy in the genomic DNA into RNA, then synthesizing DNA with the RNA as a template by reverse transcription, and inserting the DNA into the genomic DNA. A transpositionally active retrotransposon means a retrotransposon having transpositional activity. A feature of the retrotransposon is that the retrotransposon, once inserted in genomic DNA, does not change its position. Another feature thereof is that its copy number is increased on the genome. The retrotransposon is therefore classified into copy-and-paste transposons. The retrotransposon is broadly divided into LTR retrotransposons and non-LTR retrotransposons. The non-LTR retrotransposons are broadly divided into ones having a long interspersed nuclear element (LINEs), and ones having a short interspersed nuclear element (SINEs). For example, in humans, it is understood that the LTR retrotransposons account for approximately 8% of the genome, the LINEs account for approximately 21% of the genome, and the SINEs account for approximately 14% of the genome. Alu sequences are known as the SINEs to account for approximately 10% of the human genome. In humans, a sequence of the Alu family or a sequence of the L1 (LINE1) family can be preferably used. In addition, a sequence of the SINE family (e.g., an SVA sequence) or a sequence of the LTR family (e.g., an ERV(K) sequence, and an HERV-K sequence for humans) can also be preferably used. In every aspect of the present invention, the retrotransposon is preferably a transpositionally active retrotransposon. The transpositionally active retrotransposon is capable of causing polymorphic insertion.

### <Method for obtaining cell>

The present invention provides a method for obtaining a cell. In every embodiment, the cell can be preferably a cell having retrotransposons and can be a eukaryotic cell. In every embodiment, the cell can be preferably a cloned cell. In every embodiment, the method of the present invention can be preferably an *in vitro* method. Thus, in the method of the present invention, an isolated cell can be obtained. The isolated cell obtained in the present invention can be a cell having an insert of a nucleotide sequence to be inserted in a target sequence.

The cell obtained in the present invention may or may not be then cloned. In a preferred aspect, the cell obtained in the present invention can be cloned and obtained as a cloned cell. The homogeneity of the genome has the advantage that subsequent gene engineering or the like is easy to perform. In a preferred aspect, the cell obtained in the present invention is not cloned. The inhomogeneity of the genome has the advantage that the presence of a modification site is more obscure. From this viewpoint, the cell to be modified by the present invention may also be preferably an isolated cell that is not cloned. A preferred form can be appropriately selected according to a purpose as to whether or not to secure the homogeneity of the genomic DNA of the cell thus obtained, for example, whether or not to clone the cell.

In the present invention, a cell comprising nucleotide sequences of a plurality of retrotransposons is modified. In every embodiment, the retrotransposons can preferably have transpositional activity. Specifically, the present invention involves newly introducing a retrotransposon or retrotransposon-like sequence into a target sequence in genomic DNA of a cell comprising nucleotide sequences of a plurality of retrotransposons. The retrotransposon or retrotransposon-like sequence can have sequence identity (e.g., 50% or more, 60% or more, 70% or more, 80% or more, 90% or more, 95% or more, 98% or more, or 100% sequence identity) to any one of the retrotransposons in the genomic DNA. The retrotransposon or retrotransposon-like sequence may have a non-natural nucleotide sequence. The retrotransposon or retrotransposon-like sequence can be appropriately designed and synthesized by those skilled in the art. The retrotransposon or retrotransposon-like sequence can be obtained by modifying the nucleotide sequence of a retrotransposon. The modification can be nucleotide sequence modification selected from insertion, substitution, deletion, addition, and elimination. The retrotransposon or retrotransposon-like sequence can have, for example, a length within ±50%, within ±40%, within ±30%, within +20%, within ±15%, within ±10%, within ±9%, within ±8%, within ±7%, within ±6%, within ±5%, within ±4%, within ±3%, within ±2%, within ±1%, within ±10 bases, within ±9 bases, within ±8 bases, within ±7 bases, within ±6 bases, within ±5 bases, within ±4 bases, within ±3 bases, within ±2 bases, or within ±1 base of the nucleotide sequence of the retrotransposon. The retrotransposon or retrotransposon-like sequence can have the same length as that of the nucleotide sequence of the retrotransposon.

Among cells, particularly, eukaryotic cells have nucleotide sequences of a plurality (large amount) of retrotransposons in genomic DNA. Usually, the genomic DNA has inserts of retrotransposon and retrotransposon-like sequences in a large amount. In addition, these sequences voluntarily increase a copy number at a large scale and at random in the genomic DNA. These sequences increased in number and artificially inserted sequences cannot be distinguished from each other without information on artificial insertion. However, the artificial insertion can be easily identified on the basis of information on its position and sequence. For example, in random insertion, the insertion of a particular sequence to an accurate position can be stochastically ignored. For example, the probability of insertion of a particular sequence to an accurate position at a 1-base level is negligible. Thus, when there exists insertion based on information on an accurate insertion position and consistent with the information, the sequence can be estimated to be an artificially inserted sequence. By contrast, if an insertion position is unknown, whether to be a naturally occurring sequence or an artificially inserted sequence cannot be determined due to the transposon and transposon-like sequences increased in number. Thus, the presence or absence of insertion and an insertion position are not easy to identify without the basis of insertion information. It is therefore impossible to tamper an introduced label by the elimination, change, or the like of the label. Thus, the present invention can employ the modification as an impossible-to-tamper cell labeling technique.

In an aspect of the present invention, an additional transposon or transposon-like sequence can be introduced (preferably, accurately into a particular sequence with positional precision at a 1-base level) into the target sequence of the genomic DNA so as to slip into a large amount of existing retrotransposons. The introduction can be accurately performed by use of a genome modification technique such as a genome editing technique. For example, a nucleic acid sequence can be introduced to an accurate position at a 1-base level by use of a genome modification technique. By contrast, whether the introduced sequence is accurately introduced in the nucleotide sequence is easily confirmed on the basis of modification information on the nucleotide sequence of the region (e.g., information on the modified region, for example, information on the nucleotide sequence of the modified region, information on a position at which the nucleotide sequence is introduced, and information on the introduced nucleotide sequence). Retrotransposons introduce their own replicas into genomic DNA, whereas introduction positions are basically random. The probability of spontaneous and accidental insertion of a new transposon to the same position as that of the genomic DNA of the modified cell without positional difference even by one base is considered negligibly low. Therefore, when the additionally introduced transposon or transposon-like sequence is present at its introduction position with positional precision at a 1-base level (i.e., without shift even by one base), the sequence can be determined or estimated to be the artificially introduced one. However, in actuality, the insertion, deletion, or substitution of a nucleotide sequence on the order of one base to several bases can occur at a low frequency during culture. In the present specification, the term "several bases" means approximately 4 to 7 bases, preferably approximately 4 bases or 5 bases. Thus, one base to several bases mean, for example, 1 base, 2 bases, 3 bases, 4 bases, 5 bases, 6 bases, or 7 bases. Thus, in analysis, the sequence may be estimated to be inserted at a correct position even if there exists shift or difference of approximately one base to several bases. Thus, in an aspect, confirmation on whether to be inserted at a correct position further involves estimating or determining that the sequence is inserted at a correct position when shift or substitution from a designed position is approximately one base to several bases (preferably 1 to 2 bases, more preferably 1 base). This is because the probability of spontaneous and accidental insertion of a retrotransposon or retrotransposon-like sequence to a location that differs by several bases is almost negligibly low in consideration of a genome size. It is also possible to additionally introduce the same or different transposon or transposon-like sequences to a plurality of different locations (e.g., 2 to 5 locations, for example, 2 locations, or, for example, 3 locations) of the genomic DNA. Provided that the nucleotide sequences are introduced at the respective introduction locations with positional precision at a 1-base level, the sequences can be determined or estimated to be the artificially introduced ones with higher accuracy. By contrast, even the presence or absence of artificial introduction of a retrotransposon or retrotransposon-like sequence is not easy to identify without modification information on the nucleotide sequence (e.g., information on the modified region, for example, information on the nucleotide sequence of the modified region, information on a position at which the nucleotide sequence is introduced, and information on the introduced nucleotide sequence), and the position thereof is not technically easy to identify, as a matter of course. Thus, a person having no information on the introduction position can neither easily discover the nucleotide sequence of the introduction location of an artificial sequence nor tamper this sequence after discovery. Owing to this principle, the cell having genomic DNA modified by the present invention can be easily confirmed only by an engineer and a person having modification information as to whether the introduced sequence is accurately introduced in the genomic DNA. Thus, only a person having information on the nucleotide sequence of the introduction position can determine whether a test cell is a cell derived from the modified cell. On the other hand, a person having no information on the introduction position cannot identify the modification and cannot tamper the modification. In this way, the method of the present invention can be used as a cell labeling technique, while the presence or absence and position of the label can be analyzed only by an engineer and a person having modification information and are impossible to tamper. Thus, the method of the present invention is beneficial as a labeling technique. The introduction position of the additional transposon or transposon-like sequence can be a position that has no influence on cell functions such as cell survival.

The introduction of a transposon or transposon-like sequence into a target region in genomic DNA is illustrated above. However, the same holds true for the modification (nucleotide sequence modification selected from insertion, substitution, deletion, addition, and elimination) of nucleotide sequences of one or more existing transposon sequences, and the modification of neighboring sequences of existing transposon sequences as well as the change of positions of existing transposon sequences (which includes the change of relative positions to neighboring sequences). The sequences of transposons have diversity, and their neighboring sequences are also diverse. In addition, due to spontaneous large-scale increase in the number of new transposon and transposon-like sequences, it is not easy to exhaustively analyze sequences within transposons and their neighboring sequences and to identify the presence or absence of modification or the position of modification, without modification information on the modified sequence (e.g., information on the introduction position). This is because it is virtually impossible to identify artificial modification from sequences increased in number due to repeat structures of the whole transposons, their diverse nucleotide sequences, and random increase in the number thereof. On the other hand, those skilled in the art, when having information on the modified sequence, can identify a sequence within the modified transposon or its neighboring sequences on the basis of technical common sense.

Thus, the present invention provides, for example,
a method for obtaining a cell, wherein
the genomic DNA comprises nucleotide sequences of a plurality of retrotransposons,
the method comprising:
   (i) newly introducing a nucleotide sequence having 50% or more sequence identity to any one of the nucleotide sequences of the retrotransposons in the genomic DNA (i.e., a retrotransposon or retrotransposon-like nucleotide sequence) into the genomic DNA (e.g., into a target sequence thereof);
   (ii) changing an insertion position, to each neighboring sequence, of at least one of the nucleotide sequences of the retrotransposons in the genomic DNA; and/or
   (iii) modifying (nucleotide sequence modification selected from insertion, substitution, deletion, addition, and elimination) nucleotide sequences of one or more transposons of the genomic DNA.

In a preferred aspect, the present invention provides, for example,
a method for obtaining a cell, wherein
the genomic DNA comprises nucleotide sequences of a plurality of retrotransposons,
the method comprising (i) newly introducing a nucleotide sequence having 50% or more sequence identity to any one of the nucleotide sequences of the retrotransposons in the genomic DNA (i.e., a retrotransposon or retrotransposon-like nucleotide sequence) into the genomic DNA (more preferably, into a target sequence thereof).

The present invention can also provide
a cell labeling method comprising any one or more of the (i) to the (iii),
a method for obtaining a labeled cell, comprising any one or more of the (i) to the (iii), and
a method for modifying genomic DNA of a cell, comprising any one or more of the (i) to the (iii).

According to the present invention, a cell having genomic DNA having modification based on any one or more of the (i) to the (iii) can be obtained.

In the (i) to the (iii), the nucleotide sequence to be newly introduced or the modified nucleotide sequence can have sequence identity (e.g., 50% or more, 60% or more, 70% or more, 80% or more, 90% or more, 95% or more, 98% or more, or 100% sequence identity) to any of the nucleotide sequences of the retrotransposons in the genomic DNA. The nucleotide sequence to be newly introduced or the modified nucleotide sequence can have a length within ±50%, within ±40%, within ±30%, within +20%, within ±15%, within ±10%, within ±9%, within ±8%, within ±7%, within ±6%, within ±5%, within ±4%, within ±3%, within ±2%, within ±1%, within ±10 bases, within ±9 bases, within ±8 bases, within ±7 bases, within ±6 bases, within ±5 bases, within ±4 bases, within ±3 bases, within ±2 bases, or within ±1 base of any of the nucleotide sequences of the retrotransposons in the genomic DNA. The nucleotide sequence to be newly introduced or the modified nucleotide sequence can have sequence identity (e.g., 50% or more, 60% or more, 70% or more, 80% or more, 90% or more, 95% or more, 98% or more, or 100% sequence identity) to any of the nucleotide sequences of the retrotransposons in the genomic DNA and have a length within ±50%, within ±40%, within ±30%, within ±20%, within ±15%, within ±10%, within ±9%, within ±8%, within ±7%, within ±6%, within ±5%, within ±4%, within ±3%, within ±2%, within ±1%, within ±10 bases, within ±9 bases, within ±8 bases, within ±7 bases, within ±6 bases, within ±5 bases, within ±4 bases, within ±3 bases, within ±2 bases, or within ±1 base of this nucleotide sequence of the retrotransposon. The cell thus obtained by newly introducing a nucleotide sequence, changing a position of a nucleotide sequence, or newly adding modification can differ from a natural cell. By keeping the degree of the modification small, the modification is difficult to identify by a third party who does not know the contents of the modification.

In a preferred aspect, the introduction can be performed into a target sequence in the genomic DNA. The introduction of the nucleotide sequence into a target region in the genomic DNA can be performed using a genome modification system such as genome editing technique. Specifically, double-strand break is caused in a sequence-dependent manner in the target region using a genome modification system such as a genome editing technique, and the cell takes up a donor DNA sequence upon repair of the double-strand break by culture in the presence of donor DNA so that the target region can be replaced with the nucleotide sequence of the donor DNA. This step comprises introducing a genome modification system and donor DNA to the cell. The genome modification system can function within cells by introducing a component necessary therefor or introducing a polynucleotide such as DNA encoding the component necessary therefor. The donor DNA has an upstream homology arm having a nucleotide sequence homologously recombinable with an upstream nucleotide sequence of a cleavage site in the target region and a downstream homology arm having a nucleotide sequence homologously recombinable with a downstream nucleotide sequence of the cleavage site in the target region, and can comprise the retrotransposon or retrotransposon-like nucleotide sequence between the upstream homology arm and the downstream homology arm. The upstream homology arm may have a nucleotide sequence completely identical to the nucleotide sequence to be homologously recombined, or may have difference therebetween. The downstream homology arm may have a nucleotide sequence completely identical to the nucleotide sequence to be homologously recombined, or may have difference therebetween. In a preferred aspect, in the donor DNA, the upstream homology arm has a nucleotide sequence completely identical to the nucleotide sequence to be homologously recombined, the downstream homology arm has a nucleotide sequence completely identical to the nucleotide sequence to be homologously recombined, and the donor DNA may comprise the retrotransposon or retrotransposon-like nucleotide sequence between the upstream homology arm and the downstream homology arm.

In a preferred aspect, the retrotransposon or retrotransposon-like sequence can be introduced to the genomic DNA through the use of nucleotide sequence-specific cleavage and subsequent homologous recombination. In this aspect, a cell harboring the sequence can then be selected. In this context, for the purpose of efficiently selecting a cell harboring the sequence, it is preferred to select a cell that has undergone homologous recombination using a selective marker gene. In the case of introducing a selective marker gene to the genomic DNA, preferably, a portion or the whole of an artificial nucleotide sequence comprising at least the selective marker gene can be removed from the genomic DNA. This is because the possibility that the presence or absence of modification or the position of modification is identified by a third party having no modification information can be reduced by removing a larger amount of the artificial nucleotide sequence from the genomic DNA. The removal of the selective marker gene can be performed using the marker gene for negative selection as mentioned above.

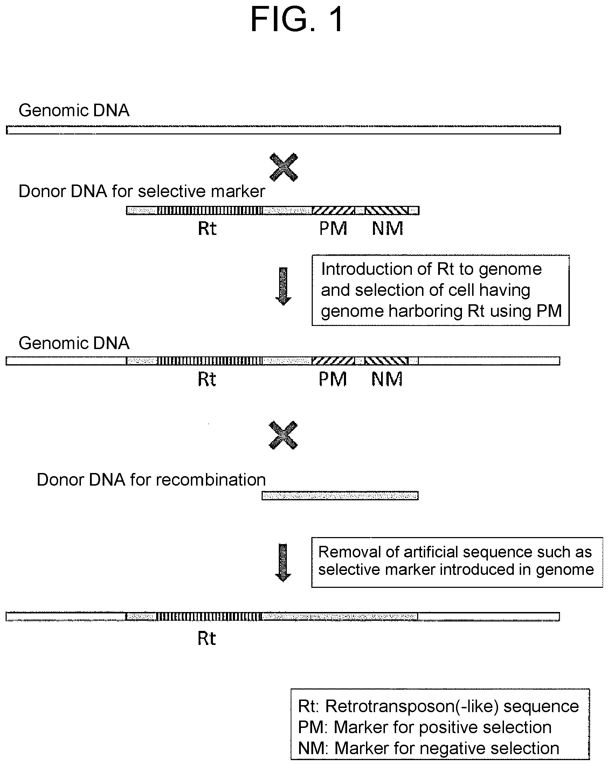

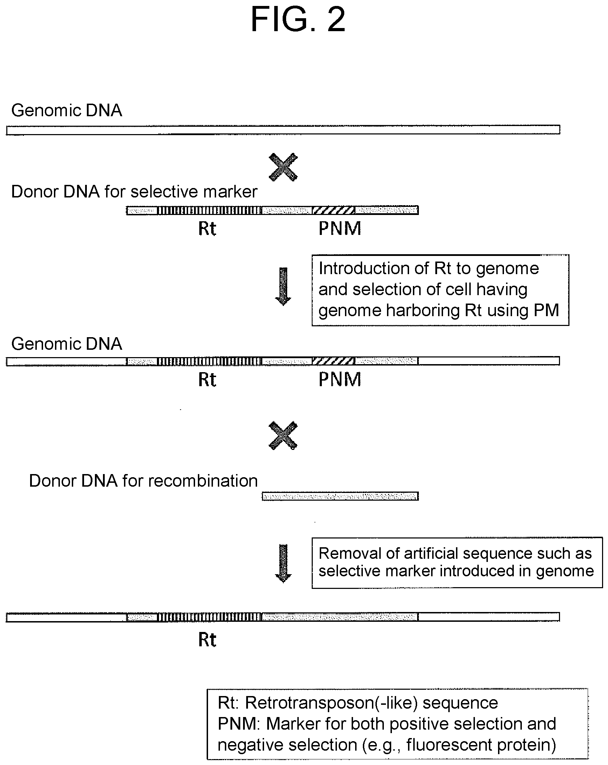

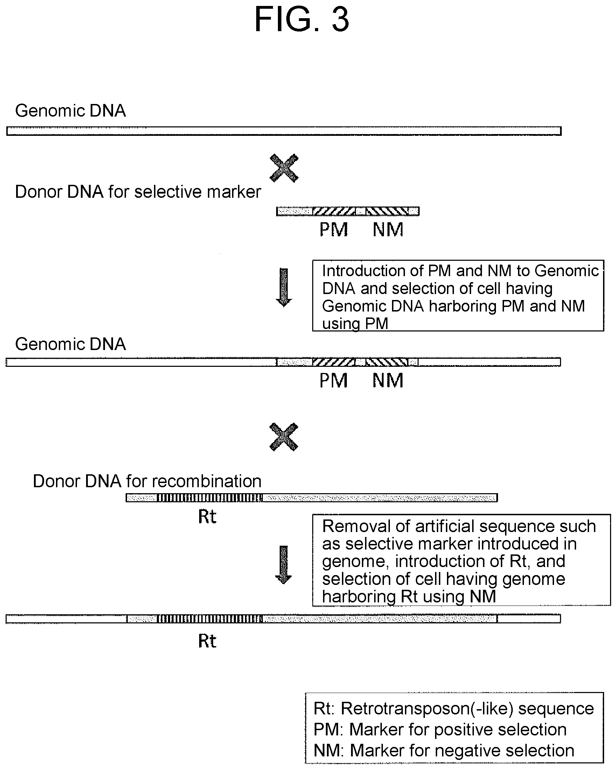

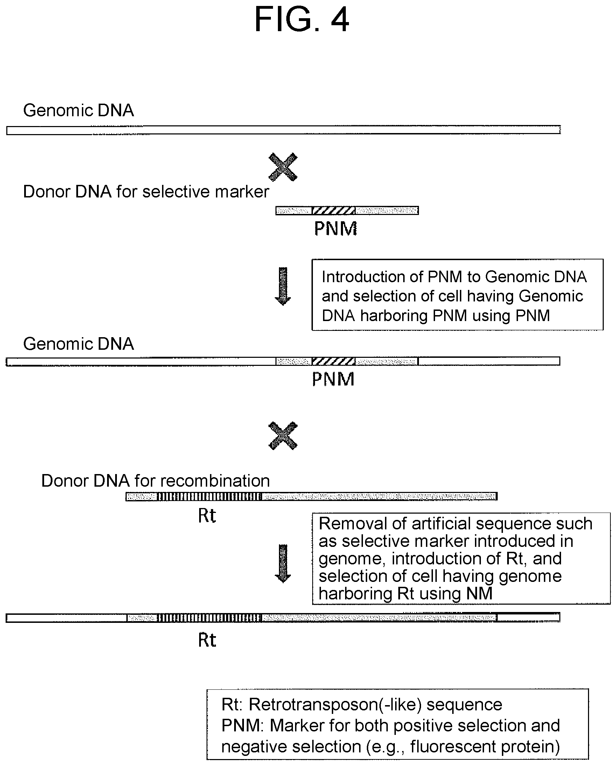

In a preferred aspect, procedures of introducing the retrotransposon or retrotransposon-like sequence are, for example, as shown in Figures 1 to 4. In these procedures, homologous recombination is caused between genomic DNA and donor DNA for a selective marker. The donor DNA for a selective marker comprises at least a marker gene for positive selection (PM) and a marker gene for negative selection (NM), or comprises a marker gene for both positive selection and negative selection (PNM). In Figures 1 and 2, the donor DNA for a selective marker has a retrotransposon sequence or retrotransposon-like sequence (Rt), and Rt is integrated in a target region of the genome at the stage described above. A cell that has undergone recombination is selected using the marker gene for positive selection. Donor DNA for recombination is allowed to act on the selected cell so that homologous recombination occurs between the genomic DNA and the donor DNA for recombination. In this respect, an artificial sequence (PM and NM or PNM) or the like introduced in the genomic DNA is removed. When recombination is successful, the marker for negative selection is no longer expressed. Therefore, such a cell can be obtained. In this way, genomic DNA with only the retrotransposon sequence or retrotransposon-like sequence (Rt) accurately inserted in the target region can be obtained. Although Figures 3 and 4 illustrate Rt loaded in the donor DNA for recombination, not in the donor DNA for a selective marker, the introduction procedures are basically the same as in Figures 1 and 2.

A preferred aspect involves introducing a marker gene for positive selection and a marker gene for negative selection (or a marker gene for both positive selection and negative selection) to the genomic DNA, and then removing the marker gene from the genomic DNA. The retrotransposon or retrotransposon-like sequence may be introduced as an adjacent sequence of the marker genes to the genomic DNA (e.g., Figures 1 and 2) or may be introduced to the genomic DNA when the marker genes are removed (e.g., Figures 3 and 4).

In a preferred aspect, the new introduction targets, for example, the target region in the chromosomal genome, and comprises: cleaving the target sequence using a genome modification system comprising a sequence-specific nucleic acid cleaving molecule capable of cleaving the target region, or a polynucleotide encoding the sequence-specific nucleic acid cleaving molecule; introducing a marker gene for positive selection and a marker gene for negative selection {wherein the marker gene for positive selection and the marker gene for negative selection may be the same gene} using donor DNA comprising an upstream homology arm having a nucleotide sequence homologously recombinable with an upstream nucleotide sequence of the cleavage site and a downstream homology arm having a nucleotide sequence homologously recombinable with a downstream nucleotide sequence of the target region; introducing the retrotransposon or retrotransposon-like sequence; and removing all the marker genes at the same time with or after the introduction of the retrotransposon or retrotransposon-like sequence.

The new insertion can comprise, for example, introducing the retrotransposon or retrotransposon-like sequence into the target sequence in the genomic DNA through the use of nucleotide sequence-specific cleavage and subsequent homologous recombination, and selecting a cell comprising the genomic DNA with the retrotransposon or retrotransposon-like sequence integrated in the target sequence.

More specifically, the new insertion can comprise, for example,
(i-1) inducing DNA repair for the target sequence in the genomic DNA by nucleotide sequence-specific cleavage of the target sequence; and introducing a marker gene for positive selection and a marker gene for negative selection or a marker gene for both positive selection and negative selection into the target region through the use of homologous recombination induced by the cleavage to select a cell harboring the marker genes, and
(i-2) then inducing DNA repair by nucleotide sequence-specific cleavage of the target sequence; and removing at least the introduced marker genes from the genomic DNA through the use of homologous recombination induced by the cleavage to select a cell lacking the marker genes, whereby
the retrotransposon or retrotransposon-like sequence is introduced, together with the marker gene, as an adjacent sequence of the marker genes to the genomic DNA, in (i-1), or introduced to the genomic DNA through the use of homologous recombination when the marker genes are removed in (i-2).

In another preferred aspect, the retrotransposon or retrotransposon-like sequence may be inserted to any non-particular site in the genomic DNA without targeting a particular insertion site. The insertion to a non-particular site can be carried out by introducing plasmid DNA for the retrotransposon or retrotransposon-like sequence to the cell. The plasmid DNA can be used, for example, to integrate the retrotransposon or retrotransposon-like sequence into the genomic DNA by transcribing the retrotransposon or retrotransposon-like sequence therefrom into RNA, and synthesizing cDNA from the transcribed RNA, for example, using a protein translated from the RNA. Thus, the plasmid DNA can have a control sequence and the retrotransposon or retrotransposon-like sequence operably linked to an control sequence. The insertion to a non-particular site may employ isolated RNA transcribed from the retrotransposon or retrotransposon-like sequence. In the case of introducing the retrotransposon or retrotransposon-like sequence to a non-particular site of the genomic DNA, information on a modified region can be obtained by extracting genomic DNA from each of the cell before the insertion and the cell after the insertion, and determining a portion or the whole of the introduction site of the retrotransposon or retrotransposon-like sequence in the genomic DNA of the cell after the insertion by comparison with the genomic DNA of the cell before the insertion. In this way, in this aspect, a cell harboring the retrotransposon or retrotransposon-like sequence and information on the modified region in the cell can be obtained. In this method, as for the determination of the introduction site, only a person who has gained the information on the modified region can determine the insertion position of the retrotransposon or retrotransposon-like sequence in the genomic DNA after the insertion, whereas it is virtually impossible for a person having no information on the modified region to determine the insertion site. Thus, a cell immediately before insertion can be stored separately from a cell after insertion, and only the cell after insertion can be used or distributed. In this aspect, the insertion site can be regarded as a target sequence.

In an aspect, the donor DNA for a selective marker comprises a target sequence between the upstream homology arm and the downstream homology arm, and comprises the retrotransposon or retrotransposon-like sequence in the target sequence. In this aspect, the target sequence can be a sequence at a location where the retrotransposon or retrotransposon-like sequence is inserted in (i) described above. Homologous recombination is caused with cleaved genomic DNA using the donor DNA designed so as to comprise the retrotransposon or retrotransposon-like sequence between a particular base and its adjacent base in the target sequence so that the sequence as designed is integrated in a site in the target sequence of the genomic DNA. (i) described above can thereby be carried out.

In an aspect, the donor DNA for a selective marker has a nucleotide sequences of at least one retrotransposon with an insertion position to a neighboring sequence changed, between the upstream homology arm and the downstream homology arm. Homologous recombination is caused with genomic DNA cleaved using the donor DNA thus designed so that the sequence as designed is integrated in a site in the target sequence of the genomic DNA. (ii) described above can thereby be carried out. The insertion position can be changed for, for example, one to several or more bases.

In an aspect, the donor DNA for a selective marker may have a modification (nucleotide sequence modification selected from insertion, substitution, deletion, addition, and elimination) in a nucleotide sequence of one or more bases to the donor DNA of the preffered aspect. (iii) can thereby be carried out. In this method, a cell in which the nucleotide sequence of a transposon at a particular position of the genomic DNA is a sequence different from the natural one can be obtained by (iii), or a cell comprising the genomic DNA having a nucleotide sequence identical to any one of the nucleotide sequences of the retrotransposons or a nucleotide sequence having 50% or more sequence identity thereto at a new position in the genomic DNA can be obtained by (i) or (ii).

For efficient modified cell obtainment, the donor DNA for a selective marker may further have a selective marker gene for positive selection instead of the retrotransposon or retrotransposon-like nucleotide sequence or in addition to the retrotransposon or retrotransposon-like nucleotide sequence. The selective marker gene for positive selection is inserted to the genomic DNA when the donor DNA is inserted. Therefore, a modified cell can be selected on the basis of the presence of the selective marker gene. When the selective marker gene is a drug resistance gene, cells can be cultured in the presence of a drug to select a modified cell while removing a non-modified cell. When the selective marker gene is a visible marker gene (e.g., a gene encoding a fluorescent protein), a cell can be selected on the basis of the expression of the marker gene to be visualized (e.g., by flow cytometry). When the donor DNA for a selective marker has the selective marker gene, the removal of the selective marker gene can be further carried out. The removal of the selective marker gene can be carried out by the application of a genome modification technique so as to replace the region with donor DNA for recombination comprising no selective marker gene. The donor DNA for recombination has an upstream homology arm having a nucleotide sequence homologously recombinable with (e.g., a nucleotide sequence completely identical to) the nucleotide sequence to be homologously recombined and a downstream homology arm having a nucleotide sequence homologously recombinable with (e.g., a nucleotide sequence completely identical to) the nucleotide sequence to be homologously recombined, and comprises the retrotransposon or retrotransposon-like nucleotide sequence between the upstream homology arm and the downstream homology arm. When the selective marker gene described above is, for example, a visible gene (e.g., a gene encoding a fluorescent protein), a cell having the modification of interest can be obtained by integrating the donor DNA for recombination to the target region by genome modification so as to replace the gene modification position of the modified cell, and selecting a cell having no selective marker on the basis of the expression of the marker gene to be visualized (e.g., by flow cytometry). When the selective marker gene is a selective marker that can be used only for positive selection, the donor DNA may further have a marker gene for negative selection. The donor DNA for recombination is integrated by genome modification so as to replace the gene modification position of the modified cell, and a cell having the modification of interest can be selected on the basis of no expression of the marker gene for negative selection (e.g., a suicide gene). When the selective marker gene is a marker gene for both positive selection and negative selection (PNM), for example, a visible gene, a cell that has undergone homologous recombination with the donor DNA for a selective marker can be selected on the basis of the presence of the visible gene while a cell that has undergone homologous recombination with the donor DNA for recombination can be selected on the basis of a loss of the visible gene.

In an aspect, the retrotransposon or retrotransposon-like sequence is introduced to at least one allele in the genomic DNA. In a preferred aspect, the retrotransposon or retrotransposon-like sequence can be similarly introduced to all alleles in the genomic DNA. Various methods well known to those skilled in the art can be used as approaches of introducing the retrotransposon or retrotransposon-like sequence to a plurality of alleles. For efficient introduction, for example, methods disclosed in International Publication No. WO 2014/093661, Ikeda et al., Nature Methods, 15: 1045-1047, 2018, and Supharattanisitthi et al., Scientific Reports, 9: 174, 2019 may be used. In an aspect, the donor DNA for a selective marker can be provided according to the number of alleles in the genomic DNA, and each donor DNA for a selective marker can be loaded with a distinguishable unique selective marker gene. A cell in which the donor DNA for a selective marker is integrated in all the alleles can be obtained on the basis of the expression (or coexpression) of all the integrated selective markers.

The genome modification system means a molecular mechanism capable of modifying a desired target region. The genome modification system comprises a sequence-specific nucleic acid cleaving molecule targeting a target region in the chromosomal genome, or a polynucleotide encoding the sequence-specific nucleic acid cleaving molecule.

The sequence-specific nucleic acid cleaving molecule is not particularly limited as long as the molecule has sequence-specific nucleic acid cleaving activity. The sequence-specific nucleic acid cleaving molecule may be a synthetic organic compound or may be a biopolymer compound such as a protein. Examples of the synthetic organic compound having sequence-specific nucleic acid cleaving activity include pyrrole-imidazole-polyamide. Examples of the protein having sequence-specific cleaving activity include sequence-specific endonuclease.

The sequence-specific endonuclease is an enzyme that can cleave a nucleic acid at a predetermined sequence. The sequence-specific endonuclease can cleave double-stranded DNA at a predetermined sequence. The sequence-specific endonuclease is not particularly limited. Examples thereof include, but are not limited to, zinc finger nuclease (ZFN), TALEN (transcription activator-like effector nuclease), and Cas protein.

ZFN is artificial nuclease containing a nucleic acid cleavage domain conjugated with a binding domain containing a zinc finger array. Examples of the cleavage domain include the cleavage domain of type II restriction enzyme FokI. Zinc finger nuclease capable of cleaving a target sequence can be designed by a method known in the art.

TALEN is artificial nuclease containing a DNA cleavage domain (e.g., a FokI domain) as well as the DNA binding domain of a transcription activator-like (TAL) effector. A TALE construct capable of cleaving a target sequence can be designed by a method known in the art (e.g., Zhang, Feng et. al. (2011) Nature Biotechnology 29 (2)).

In the case of using Cas protein as the sequence-specific nucleic acid cleaving molecule, the genome modification system comprises a CRISPR/Cas system. Specifically, the genome modification system preferably comprises Cas protein, and guide RNA having a nucleotide sequence homologous to a nucleotide sequence within the target region. The guide RNA can comprise, as a spacer sequence, a sequence homologous to a sequence (target sequence) within the target region. The guide RNA can bind to DNA within the target region and does not have to have a sequence completely identical to the target sequence. This binding can be formed under physiological conditions in the cell nucleus. The guide RNA can contain, for example, 0- to 3-base mismatches with respect to the target sequence. The number of the mismatches is preferably 0 to 2 bases, more preferably 0 to 1 bases, further preferably zero mismatch. The guide RNA can be designed on the basis of a method known in the art. The genome modification system is preferably a CRISPR/Cas system and preferably comprises Cas protein and guide RNA. The Cas protein is preferably Cas9 protein.

The sequence-specific endonuclease may be introduced as a protein to the cell, or may be introduced as a polynucleotide encoding the sequence-specific endonuclease to the cell. For example, mRNA of the sequence-specific endonuclease may be introduced, or an expression vector of the sequence-specific endonuclease may be introduced. In the expression vector, a coding sequence (sequence-specific endonuclease gene) of the sequence-specific endonuclease is functionally linked to a promoter. The promoter is not particularly limited, and, for example, various pol II promoters can be used. Examples of the pol II promoter include, but are not particularly limited to, CMV promoter, EF1 promoter (EF1α promoter), SV40 promoter, MSCV promoter, hTERT promoter, β actin promoter, CAG promoter, and CBh promoter.

The promoter may be an inducible promoter. The inducible promoter is a promoter that can induce the expression of a polynucleotide functionally linked to this promoter in the presence of an inducer that drives the promoter. Examples of the inducible promoter include promoters, such as heat shock promoter, which induce gene expression by heating. Other examples of the inducible promoter include promoters for which the inducer that drives the promoter is a drug. Examples of such a drug-inducible promoter include Cumate operator sequences, À operator sequences (e.g., 12 × λOp), and tetracycline-inducible promoter. Examples of the tetracycline-inducible promoter include promoters that drive gene expression in the presence of tetracycline or a derivative thereof (e.g., doxycycline), or reverse tetracycline-controlled transactivator (rtTA). Examples of the tetracycline-inducible promoter include TRE3G promoter.

An expression vector known in the art can be used without particular limitations. Examples of the expression vector include plasmid vectors and virus vectors. When the sequence-specific endonuclease is Cas protein, the expression vector may contain a coding sequence (Cas protein gene) of the Cas protein as well as a guide RNA coding sequence (guide RNA gene). In this case, it is preferred that the guide RNA coding sequence (guide RNA gene) should be functionally linked to pol III promoter. Examples of the pol III promoter include mouse and human U6-snRNA promoters, human H1-RNase P RNA promoter, and human valine-tRNA promoter. The donor DNAs are donor DNAs for knocking-in selective markers to target regions. Each of the donor DNAs for selective markers comprises the nucleotide sequences of one or more selective marker genes between an upstream homology arm having a nucleotide sequence homologous to an upstream nucleotide sequence adjacent to the target region and a downstream homology arm having a nucleotide sequence homologous to a downstream nucleotide sequence adjacent to the target region.

The donor DNAs can have a length of, for example, but not particularly limited to, 1 kb or more, 2 kb or more, 3 kb or more, 4 kb or more, 5 kb or more, 6 kb or more, 7 kb or more, 8 kb or more, 9 kb or more, 9.5 kb or more, or 10 kb or more. The donor DNAs for selective markers can have a length of, for example, but not particularly limited to, 50 kb or less, 45 kb or less, 40 kb or less, 35 kb or less, 30 kb or less, 25 kb or less, 20 kb or less, 15 kb or less, 14 kb or less, 13 kb or less, 12 kb or less, 11 kb or less, 10 kb or less, 9 kb or less, 8 kb or less, 7 kb or less, 6 kb or less, 5 kb or less, or 4 kb or less.

The "selective marker" means a protein that permits cells to be selected on the basis of the presence or absence of its expression. The selective marker gene is a gene encoding the selective marker. In the case of selecting selective marker-expressing cells from a cell population in which the selective marker-expressing cells coexist with selective marker non-expressing cells, the selective marker is referred to as a "positive selective marker" or a "selective marker for positive selection". In the case of selecting selective marker non-expressing cells from a cell population in which selective marker-expressing cells coexist with the selective marker non-expressing cells, the selective marker is referred to as a "negative selective marker" or a "selective marker for negative selection". Different selective markers mean that the selective markers can be distinguished from each other (e.g., distinguishably different), and means that, for example, the selective markers can be distinguished from each other at least in terms of physiological properties such as drug resistance property or other physicochemical properties imparted to cells harboring the selective markers.

The positive selective marker is not particularly limited as long as a cell expressing the positive selective marker can be selected. Examples of the positive selective marker gene include drug resistance genes, fluorescent protein genes, luminescent enzyme genes, and chromogenic enzyme genes.

The negative selective marker is not particularly limited as long as a cell not expressing the negative selective marker can be selected. Examples of the negative selective marker gene include suicide genes (thymidine kinase, etc.), fluorescent protein genes, luminescent enzyme genes, and chromogenic enzyme genes. When the negative selective marker gene is a gene that has negative influence on the survival of cells (e.g., a suicide gene), the negative selective marker gene can be functionally linked to an inducible promoter. The negative selective marker gene thus functionally linked to the inducible promoter can be expressed only when the removal of cells having the negative selective marker gene is desired. The negative selective marker gene, for example, an optically detectable (e.g., fluorescent, luminescent, and chromogenic) marker gene (visible marker gene), may be constitutively expressed because of having little negative influence on the survival of cells.

Examples of the drug resistance gene include, but are not limited to, puromycin resistance gene, blasticidin resistance gene, geneticin resistance gene, neomycin resistance gene, tetracycline resistance gene, kanamycin resistance gene, zeocin resistance gene, hygromycin resistance gene, and chloramphenicol resistance gene.

Examples of the fluorescent protein gene include, but are not limited to, green fluorescent protein (GFP) gene, yellow fluorescent protein (YFP) gene, and red fluorescent protein (RFP) gene.

Examples of the luminescent enzyme gene include, but are not limited to, luciferase gene.

Examples of the chromogenic enzyme gene include, but are not limited to, β galactosidase gene, β glucuronidase gene, and alkaline phosphatase gene.

Examples of the suicide gene include, but are not limited to, herpes simplex virus thymidine kinase (HSV-TK) and inducible caspase 9.

The selective marker genes carried by the donor DNAs for selective markers are preferably positive selective marker genes. Specifically, cells expressing the selective markers can be selected as cells in which the selective marker genes are knocked in.

The upstream homology arm has a nucleotide sequence homologously recombinable with an upstream nucleotide sequence of a cleavage site in the target region in the genome to be modified, and has, for example, a nucleotide sequence homologous to an upstream nucleotide sequence adjacent to a cleavage site in the target sequence. The downstream homology arm has a nucleotide sequence homologously recombinable with a downstream nucleotide sequence of a cleavage site in the target region in the genome to be modified, and has, for example, a nucleotide sequence homologous to a downstream nucleotide sequence adjacent to a cleavage site in the target sequence. The upstream homology arm and the downstream homology arm are not particularly limited by their lengths and sequences as long as these homology arms are homologously recombinable with the neighboring regions of the target region. The upstream homology arm and the downstream homology arm are not necessarily required to be identical to the upstream sequence and the downstream sequence, respectively, of the target region as long as these homology arms are homologously recombinable therewith. For example, the upstream homology arm can be a sequence having 90% or more sequence identity (homology) to the upstream nucleotide sequence adjacent to the target region and preferably has 92% or more, 93% or more, 94% or more, 95% or more, 96% or more, 97% or more, 98% or more, or 99% or more sequence identity thereto. For example, the downstream homology arm can be a sequence having 90% or more sequence identity (homology) to the downstream nucleotide sequence adjacent to the target region and preferably has 92% or more, 93% or more, 94% or more, 95% or more, 96% or more, 97% or more, 98% or more, or 99% or more sequence identity thereto. The modification efficiency of the alleles can be enhanced provided that at least any one of the upstream homology arm and the downstream homology arm is located closer to a cleavage location in or near the target region. In this context, the term "close" can mean that the distance between two sequences is 100 bp or less, 50 bp or less, 40 bp or less, 30 bp or less, 20 bp or less, or 10 bp or less.

In the donor DNAs for selective markers, the selective marker gene is positioned between the upstream homology arm and the downstream homology arm. As a result, in the case of introducing the donor DNAs for selective markers, together with the genome modification system (i), to the cell, the selective marker gene is introduced to the target region by HDR (this is referred to gene knock-out when the gene is disrupted, and referred to as gene knock-in when the desired gene is introduced; a gene may be knocked out while another gene can be knocked in).

It is preferred that the selective marker gene should be functionally linked to a promoter so as to be expressed under the control of an appropriate promoter. The promoter can be appropriately selected according to the type of the cell to which the donor DNAs are to be introduced. Examples of the promoter include SRα promoter, SV40 early promoter, retrovirus LTR, CMV (cytomegalovirus) promoter, RSV (Rous sarcoma virus) promoter, HSV-TK (herpes simplex virus thymidine kinase) promoter, EF1α promoter, metallothionein promoter, and heat shock promoter. Each of the donor DNAs for selective markers may have, for example, an arbitrary control sequence such as an enhancer, a poly-A addition signal, or a terminator.

Each of the donor DNAs for selective markers may have an insulator sequence. The "insulator" refers to a sequence that ensures or enhances the independence of transcriptional regulation of DNA flanked by its regions by blocking or mitigating the influence of adjacent chromosomal environments. The insulator is defined by an enhancer blocking effect (effect of blocking the influence of an enhancer on promoter activity by the insulator inserted between the enhancer and the promoter), and a suppressive effect on a position effect (effect of preventing the expression of a transgene from being influenced by the position of the insert in the genome, by the insulators flanking both sides of the transgene). Each of the donor DNAs for selective markers may have an insulator sequence between the upstream arm and the selective marker gene (or between the upstream arm and a promoter that controls the selective marker gene). Each of the donor DNAs for selective markers may have an insulator sequence between the downstream arm and the selective marker gene.

The donor DNAs for selective markers may be linear or may be cyclic and are preferably cyclic. Preferably, the donor DNAs for selective markers are plasmids. Each of the donor DNAs for selective markers may comprise an arbitrary sequence in addition to the sequences described above. For example, a spacer sequence may be contained wholly or partially between the respective sequences of the upstream homology arm, the insulator, the selective marker gene, and the downstream homology arm.

A method for introducing the genome modification system, the donor DNA for a selective marker, and the donor DNA for recombination to the cell is not particularly limited, and a method known in the art can be used without particular limitations. Examples of the method for introducing the genome modification system, the donor DNA, and the donor DNA for recombination to the cell include, but are not limited to, viral infection method, lipofection method, microinjection method, calcium phosphate method, DEAE-dextran method, electroporation method, and particle gun method.

In an aspect, each of the donor DNAs for selective markers has an upstream homology arm and a downstream homology arm and has a selective marker gene between the upstream homology arm and the downstream homology arm, and preferably, may further have a target sequence of endonuclease (nucleotide sequence-specific nucleic acid cleaving molecule), such as a cleavage site of meganuclease. In a preferred aspect of this aspect, the selective markers include selective marker genes for positive selections and a marker gene for negative selection. In another preferred aspect, the selective markers include selective markers for positive selections but may not include a negative selective marker gene aside therefrom. In a preferred aspect, the selective marker gene for positive selection may also be used for negative selection. Examples of such a marker gene include visible marker genes.

Since the target region of the genomic DNA to be modified is replaced with the sequences of the donor DNA for a selective marker and the donor DNA for recombination, modification in any of (i) to (iii) described above can be carried out by designing the replacing nucleotide sequence so as to attain any of (i) to (iii) described above. Only any one of (i) to (iii) described above may be carried out, or (i) to (iii) may be carried out in combination. Specifically, (i) may be carried out as to a transposon sequence or transposons-like sequence, and (i) can also be carried out as to another transposon sequence or transposons-like sequence. (ii) may be carried out as to a transposon sequence or transposons-like sequence, and (ii) can also be carried out as to another transposon sequence or transposons-like sequence. (iii) may be carried out as to a transposon sequence or transposons-like sequence, and (iii) can also be carried out as to another transposon sequence or transposons-like sequence. (i) may be carried out as to a transposon sequence or transposons-like sequence, and (ii) can also be carried out as to another transposon sequence or transposons-like sequence. (i) may be carried out as to a transposon sequence or transposons-like sequence, and (iii) can also be carried out as to another transposon sequence or transposons-like sequence. (ii) may be carried out as to a transposon sequence or transposons-like sequence, and (iii) can also be carried out as to another transposon sequence or transposons-like sequence.

In a preferred aspect, the retrotransposons are LINEs. In a preferred aspect, the retrotransposons are SINEs. In a preferred aspect, the retrotransposons are Alus. In a preferred aspect, the retrotransposons are LTR retrotransposons. In a preferred aspect, a sequence of the Alu family or a sequence of the L1 (LINE1) family can be preferably used as a human retrotransposon. In addition, a sequence of the SINE family (e.g., an SVA sequence) or a sequence of the LTR family (e.g., an ERV(K) sequence, and an HERV-K sequence for humans) can also be preferably used. In every aspect of the present invention, the retrotransposons are preferably transpositionally active retrotransposons. These retrotransposons have numerous copies on the genome and are therefore preferred because the retrotransposons increase the difficulty in identifying a modification site by a third party who does not know positional information on the modification site. In an aspect, retrotransposons with the highest frequency of occurrence (or at least the second, third, fourth, or fifth highest frequency of occurrence) in the genome of the organism species can be used. The retrotransposons are not limited to those described above and differ depending on an organism species, and retrotransposons having more copies in the genome can be more preferably used in the present invention.

### <Engineered cell (i) of present invention>

The present invention provides
a cell having, in a target sequence in genomic DNA, an additional nucleotide sequence having 50% or more sequence identity to any one of nucleotide sequences of retrotransposons in the genomic DNA.

In an aspect, the additional nucleotide sequence can have sequence identity (e.g., 50% or more, 60% or more, 70% or more, 80% or more, 90% or more, 95% or more, 98% or more, or 100% sequence identity) to any one of the nucleotide sequences of the retrotransposons in the genomic DNA. In an aspect, the additional nucleotide sequence has a length within ±50%, within ±40%, within +30%, within ±20%, within ±15%, within ±10%, within ±9%, within ±8%, within ±7%, within ±6%, within ±5%, within ±4%, within ±3%, within ±2%, within ±1%, within ±10 bases, within ±9 bases, within ±8 bases, within ±7 bases, within ±6 bases, within ±5 bases, within ±4 bases, within ±3 bases, within ±2 bases, or within ±1 base of any one of the nucleotide sequences of the retrotransposons in the genomic DNA.

According to the present invention, the cell may further have, in a second target sequence in the genomic DNA, a second additional nucleotide sequence having 50% or more sequence identity to any one of the nucleotide sequences of the retrotransposons in the genomic DNA, in addition to the additional nucleotide sequence (first nucleotide sequence). In an aspect, the second nucleotide sequence can have sequence identity (e.g., 50% or more, 60% or more, 70% or more, 80% or more, 90% or more, 95% or more, 98% or more, or 100% sequence identity) to any one of the nucleotide sequences of the retrotransposons in the genomic DNA. In an aspect, the second nucleotide sequence has a length within ±50%, within ±40%, within ±30%, within ±20%, within ±15%, within ±10%, within ±9%, within ±8%, within ±7%, within ±6%, within ±5%, within ±4%, within ±3%, within ±2%, within ±1%, within ±10 bases, within ±9 bases, within ±8 bases, within ±7 bases, within ±6 bases, within ±5 bases, within ±4 bases, within ±3 bases, within ±2 bases, or within ±1 base of any one of the nucleotide sequences of the retrotransposons in the genomic DNA. In an aspect, the first nucleotide sequence and the second nucleotide sequence are the same. In an aspect, the first nucleotide sequence and the second nucleotide sequence are different.

### <Engineered cell (ii) of present invention>

The present invention provides
a cell in which an insertion position of at least one of nucleotide sequences of retrotransposons in genomic DNA to a neighboring sequence has been changed.

The present invention also provides
a cell having a nucleotide sequence of at least one retrotransposon with an insertion position to a neighboring sequence changed in genomic DNA.

### <Engineered cell (iii) of present invention>

The present invention provides
a cell comprising a nucleotide sequence of a modified transposon.

The modification can be nucleotide sequence modification selected from insertion, substitution, deletion, addition, and elimination. The modification can be performed inside the nucleotide sequence of a transposon. However, the addition and the elimination are addition and elimination, respectively, at an end of the nucleotide sequence of a transposon. The modification causes the change of the original sequence to a different sequence, and the different sequence can be, for example, an artificial sequence (e.g., a non-natural sequence) or can be a natural different sequence.

According to the present invention, the modified cells (i) to (iii) of the present invention have neither a selective marker gene nor a site-specific recombination sequence (e.g., loxP) at the modification site.

The present invention provides a cell population comprising any one or more of the modified cells (i) to (iii) of the present invention. The present invention provides a composition comprising any one or more of the modified cells (i) to (iii) of the present invention. The composition can have conditions suitable for the survival or preservation of the cell. The cell, the cell population, and the composition comprising the cell may be frozen. The freezing can be performed in, for example, liquid nitrogen. Cryopreservation can be performed in liquid nitrogen or in a deep freezer (e.g., having a temperature of approximately -80°C). The modified cells (i) to (iii) of the present invention can be isolated cells. The cell population can be a population of isolated cells. In an aspect, the present invention provides a combination of any of the modified cells (i) to (iii) of the present invention and information on a modified region thereof (e.g., one or more pieces of information selected from the group consisting of information on the nucleotide sequence of the modified region, information on a sequence insertion position, and information on the inserted nucleotide sequence). The combination can be used in, for example, a cell management method and management system as well as a cell analysis method mentioned later.

The present invention provides genomic DNA of any one or more of the modified cells (i) to (iii) of the present invention. The genomic DNA can be isolated genomic DNA. The isolated genomic DNA can be in a form dissolved in an aqueous solution, a frozen form, or a dried form. In an aspect, the present invention provides a combination of isolated genomic DNA of any of the modified cells (i) to (iii) of the present invention and information on a modified region thereof. The combination can be used in, for example, a cell management method and management system as well as a cell analysis method mentioned later.

### <Cell management method and management system>

According to the present invention, the modified cells (i) to (iii) of the present invention can be cultured, stored, or managed in connection with information on modification (particularly, positional information on the modification site and/or information on the sequence thus modified). The present invention provides, for example, a container containing any of the modified cells (i) to (iii) of the present invention, and the container comprises a label. The label can be readable information, for example, readable information (e.g., printing) selected from a letter string, a barcode (including a two-dimensional barcode), and RFID, and the type of the modification made in the cell contained in the container can be determined by reading the information, and checking the information against a stored record of the modification information. In a preferred aspect, the readable information differs among containers and corresponds to the container on a one-to-one basis.

The present invention provides a cell management system comprising:
a recording medium (e.g., a non-volatile memory) that stores readable information corresponding to each of a plurality of containers each containing any of the modified cells (i) to (iii) of the present invention, and a correspondence table comprising modification information on each cell associated with the readable information;
a reception part (e.g., a volatile memory) that receives the readable information; and
a processor that executes a program to identify modification information on the cell contained in the container having the received readable information on the basis of the correspondence table read from the recording medium.

The present invention provides a cell management system comprising:
a recording medium (e.g., a non-volatile memory) that stores readable information corresponding to each of a plurality of containers each containing any of the modified cells (i) to (iii) of the present invention, and a correspondence table comprising modification information on each cell associated with the readable information;
a reception part that receives the cell modification information; and
a processor that executes a program to identify a container given the readable information corresponding to modification information of the received cell on the basis of the correspondence table read from the recording medium.

The present invention provides a method for managing any of the modified cells (i) to (iii) of the present invention, the method comprising:
providing a plurality of containers each containing any of the modified cells (i) to (iii) of the present invention, the containers being each given readable information;
providing readable information corresponding to each of the plurality of containers each containing any of the modified cells (i) to (iii) of the present invention, and a correspondence table comprising information on a modified region of each cell associated with the readable information; and
identifying information on a modified region in the cell contained in the container from the readable information given to the container on the basis of the correspondence table, and/or identifying a container containing a cell having the modification from the information on the cell modified region.

In this aspect, the method of the present invention can be carried out using the cell management system of the present invention.

According to the present invention, the cell management system comprises containers containing one or more modified cells of the present invention. The containers can comprise readable information corresponding to each of the containers, and the cell management system can comprise a correspondence table comprising information on a modified region of each cell associated with the readable information. Thus, the present invention provides a cell management system comprising containers containing one or more modified cells of the present invention, wherein the containers comprise readable information corresponding to each of the containers, and comprise a correspondence table comprising information on a modified region of each cell associated with the readable information. In the cell management system, a portion or the whole of the one or more modified cells of the present invention may be in a cryopreserved state, or a portion or the whole of the one or more modified cells of the present invention may be being cultured. The modified cells of the present invention can be the modified cells (i) to (iii) described above.

The present invention can provide a management system of genomic DNA of the cell or a modified region-containing fragment of the genomic DNA. This management system is the same as that described above except that the genomic DNA of the cell or the modified region-containing fragment of the genomic DNA is used instead of the cell in the cell management system described above. Thus, the "cell" in the cell management system described above can be read as the "genomic DNA of the cell or the modified region-containing fragment of the genomic DNA".

Specifically, according to the present invention, the genomic DNA of any of the modified cells (i) to (iii) of the present invention or the modified region-containing fragment of the genomic DNA, or information thereon can be stored or managed in connection with information on modification (particularly, positional information on the modification site and/or information on the sequence thus modified). The present invention provides, for example, a container containing genomic DNA of any of the modified cells (i) to (iii) of the present invention or a modified region-containing fragment of the genomic DNA, and the container comprises a label. The label can be readable information, for example, readable information (e.g., printing) selected from a letter string, a barcode (including a two-dimensional barcode), and RFID, and the type of the modification made in the cell from which the genomic DNA or the modified region-containing fragment of the genomic DNA contained in the container is derived can be determined by reading the information, and checking the information against a stored record of the modification information. In a preferred aspect, the readable information differs among containers and corresponds to the container on a one-to-one basis.

The present invention provides a management system of genomic DNA or a modified region-containing fragment of the genomic DNA, the management system comprising:
a recording medium (e.g., a non-volatile memory) that stores readable information corresponding to each of a plurality of containers each containing genomic DNA of any of the modified cells (i) to (iii) of the present invention or a modified region-containing fragment of the genomic DNA, and a correspondence table comprising modification information on each cell associated with the readable information;
a reception part (e.g., a volatile memory) that receives the readable information; and
a processor that executes a program to identify modification information on the genomic DNA or the modified region-containing fragment of the genomic DNA contained in the container having the received readable information on the basis of the correspondence table read from the recording medium.

The present invention provides a management system of genomic DNA or a modified region-containing fragment of the genomic DNA, the management system comprising:
a recording medium (e.g., a non-volatile memory) that stores readable information corresponding to each of a plurality of containers each containing genomic DNA of any of the modified cells (i) to (iii) of the present invention or a modified region-containing fragment of the genomic DNA, and a correspondence table comprising modification information on each genomic DNA or modified region-containing fragment of the genomic DNA associated with the readable information;
a reception part that receives the modification information on the genomic DNA or the modified region-containing fragment of the genomic DNA; and
a processor that executes a program to identify a container given the readable information corresponding to the received modification information on the genomic DNA or the modified region-containing fragment of the genomic DNA on the basis of the correspondence table read from the recording medium.

The present invention provides a method for managing genomic DNA of any of the modified cells (i) to (iii) of the present invention or a modified region-containing fragment of the genomic DNA, the method comprising:
providing a plurality of containers each containing the genomic DNA of any of the modified cells (i) to (iii) of the present invention or the modified region-containing fragment of the genomic DNA, the containers being each given readable information;
providing readable information corresponding to each of the plurality of containers each containing the genomic DNA of any of the modified cells (i) to (iii) of the present invention or the modified region-containing fragment of the genomic DNA, and a correspondence table comprising information on a modified region of each genomic DNA or modified region-containing fragment of the genomic DNA associated with the readable information; and
identifying information on a modified region in the genomic DNA or the modified region-containing fragment of the genomic DNA contained in the container from the readable information given to the container on the basis of the correspondence table, and/or identifying genomic DNA or a modified region-containing fragment of the genomic DNA having the modification or a container containing it from the information on the modified region in the genomic DNA or the modified region-containing fragment of the genomic DNA.

In this aspect, the method of the present invention can be carried out using the management system of genomic DNA or a modified region-containing fragment of the genomic DNA of the present invention.

According to the present invention, the management system of genomic DNA or a modified region-containing fragment of the genomic DNA comprises containers containing genomic DNAs of one or more modified cells of the present invention or modified region-containing fragments of the genomic DNAs. The containers can comprise readable information corresponding to each of the containers, and the management system can comprise a correspondence table comprising information on a modified region of each genomic DNA or modified region-containing fragment of the genomic DNA associated with the readable information. Thus, the present invention provides a management system comprising containers containing genomic DNAs of one or more modified cells of the present invention or modified region-containing fragments of the genomic DNAs, wherein the containers comprise readable information corresponding to each of the containers, and comprise a correspondence table comprising information on a modified region of each genomic DNA or modified region-containing fragment of the genomic DNA associated with the readable information. In the management system of genomic DNA or a modified region-containing fragment of the genomic DNA, a portion or the whole of the genomic DNAs of one or more modified cells of the present invention or the modified region-containing fragments of the genomic DNAs may be in a cryopreserved state. The genomic DNAs of the modified cells of the present invention or the modified region-containing fragments of the genomic DNAs can be genomic DNAs of the modified cells (i) to (iii) described above or modified region-containing fragments of the genomic DNAs.

### <Method for analyzing cell modification by present invention>

According to the present invention, the modified cells (i) to (iii) of the present invention, cells derived from the modified cells (i) to (iii) of the present invention, or cells having the possibility of being the modified cells (i) to (iii) of the present invention can be analyzed on the basis of the nucleotide sequence of a modified region in the cells and information on the modified region (particularly, positional information on the modification site and/or information on the sequence thus modified, particularly, positional information on the modification site). According to the present invention, provided that information on the modified region, for example, positional information on the modification site, is understood, whether or not cells are the modified cells (i) to (iii) of the present invention can be determined or estimated by decoding the nucleotide sequence of the site. The cells derived from the modified cells (i) to (iii) of the present invention can be cells obtained by the proliferation of the cells or cells obtained by adding another gene modification to the cells. The cells derived from the modified cells (i) to (iii) of the present invention have the modification of the present invention in the modified region unless the modification site is tampered. Therefore, in the present invention, such cells can be used for determining or estimating modified cells. Furthermore, it is virtually impossible to identify and tamper the modification, as described above.

For example, when the retrotransposon or retrotransposon-like sequence is determined or estimated to be inserted (or present) in the modified region, the cells can be determined or estimated to be the modified cells (i) to (iii) of the present invention or cells derived therefrom, or possibly these cells. Thus, the present invention may involve
(α) when the retrotransposon or retrotransposon-like sequence is inserted (or present) in the modified region (particularly, when the retrotransposon or retrotransposon-like sequence is determined or estimated to be inserted (or present) in the modified region), determining or estimating that the cells are the modified cells (i) to (iii) of the present invention or cells derived therefrom, or possibly these cells.

When the retrotransposon or retrotransposon-like sequence is not inserted (or not present) in the modified region (particularly, when the retrotransposon or retrotransposon-like sequence is determined or estimated to be not inserted (or not present) in the modified region), the cells can be determined or estimated to be not the modified cells (i) to (iii) of the present invention or cells derived therefrom, or possibly not these cells. If cells have undergone modification at a plurality of sites, similar analysis can be carried out as to a portion or the whole thereof. Thus, the present invention may involve
(β) when the retrotransposon or retrotransposon-like sequence is not inserted (or not present) in the modified region (particularly, when the retrotransposon or retrotransposon-like sequence is determined or estimated to be not inserted (or not present) in the modified region), determining or estimating that the cells are not the modified cells (i) to (iii) of the present invention or cells derived therefrom, or possibly not these cells.

The present invention may involve any one of the (α) and the (β) and may involve both the (α) and the (β). Specifically, the present invention may involve: when the retrotransposon or retrotransposon-like sequence is inserted (or present) in the modified region (particularly, when the retrotransposon or retrotransposon-like sequence is determined to be inserted (or present) in the modified region), determining or estimating that the cells are the modified cells (i) to (iii) of the present invention or cells derived therefrom, or possibly these cells; and/or when the retrotransposon or retrotransposon-like sequence is not inserted (or not present) in the modified region (particularly, when the retrotransposon or retrotransposon-like sequence is determined or estimated to be not inserted (or not present) in the modified region), determining or estimating that the cells are not the modified cells (i) to (iii) of the present invention or cells derived therefrom, or possibly not these cells. When modification at all of a plurality of sites is determined or estimated to be modification consistent with the information on modification, this means that the analyzed cells are determined or estimated to be the modified cells (i) to (iii) of the present invention with higher precision. In this context, the "modified region" is a region having an insert of a nucleotide sequence or a positionally changed nucleotide sequence. The modified region comprises an inserted sequence and one base each on both sides or consists of an inserted sequence and one base each on both sides. The modified region may comprise an inserted sequence and one to several bases each on both sides or consist of an inserted sequence and one to several bases each on both sides. A mutation such as insertion or deletion may occur in the genome, as mentioned later, when cells are cultured over a long period in such a way that the cells are subjected to repetitive passages. In such a case, the cells may be determined or estimated to be derived from the modified cells even if there is shift from an insertion position, in consideration of a wider modified region.

The present invention can provide a method for analyzing genomic DNA of the cell or a modified region-containing fragment of the genomic DNA. This method is the same as the cell analysis method described above except that the genomic DNA of the cell or the modified region-containing fragment of the genomic DNA is used instead of the cell in the cell analysis method described above. Thus, the "cell" in the cell analysis method described above can be read as the "genomic DNA of the cell or the modified region-containing fragment of the genomic DNA". Specifically, according to the present invention, genomic DNAs of the modified cells (i) to (iii) of the present invention, cells derived from the modified cells (i) to (iii) of the present invention, or cells having the possibility of being the modified cells (i) to (iii) of the present invention or modified region-containing fragments of the genomic DNAs can be analyzed on the basis of the nucleotide sequence of a modified region in the genomic DNAs or the modified region-containing fragments of the genomic DNAs and information on the modified region (particularly, positional information on the modification site and/or information on the sequence thus modified, particularly, positional information on the modification site). According to the present invention, provided that information on the modified region, for example, positional information on the modification site, is understood, whether or not genomic DNAs or modified region-containing fragments of the genomic DNAs are the genomic DNAs of the modified cells (i) to (iii) of the present invention or the modified region-containing fragments of the genomic DNAs can be determined or estimated by decoding the nucleotide sequence of the site. The cells derived from the modified cells (i) to (iii) of the present invention can be cells obtained by the proliferation of the cells or cells obtained by adding another gene modification to the cells. The genomic DNAs of the cells derived from the modified cells (i) to (iii) of the present invention or the modified region-containing fragments of the genomic DNAs have the modification of the present invention in the modified region unless the modification site is tampered. Therefore, in the present invention, such genomic DNAs or fragments can be used for determining or estimating genomic DNAs of modified cells or modified region-containing fragments of the genomic DNAs. Furthermore, it is virtually impossible to identify and tamper the modification, as described above.

For example, when the retrotransposon or retrotransposon-like sequence is determined or estimated to be inserted (or present) in the modified region, the genomic DNAs or the modified region-containing fragments of the genomic DNAs can be determined or estimated to be genomic DNAs of the modified cells (i) to (iii) of the present invention or cells derived therefrom or modified region-containing fragments of the genomic DNAs, or possibly these genomic DNAs or fragments. Thus, the present invention may involve
(α) when the retrotransposon or retrotransposon-like sequence is inserted (or present) in the modified region (particularly, when the retrotransposon or retrotransposon-like sequence is determined or estimated to be inserted (or present) in the modified region), determining or estimating that the cells are genomic DNAs of the modified cells (i) to (iii) of the present invention or cells derived therefrom or modified region-containing fragments of the genomic DNAs, or possibly these genomic DNAs or fragments.

When the retrotransposon or retrotransposon-like sequence is not inserted (or not present) in the modified region (particularly, when the retrotransposon or retrotransposon-like sequence is determined or estimated to be inserted (or present) in the modified region), the genomic DNAs or the engineered region-containing fragments of the genomic DNAs can be determined or estimated to be not genomic DNAs of the modified cells (i) to (iii) of the present invention or cells derived therefrom or modified region-containing fragments of the genomic DNAs, or possibly not these genomic DNAs or fragments. If cells have undergone modification at a plurality of sites, similar analysis can be carried out as to a portion or the whole thereof. Thus, the present invention may involve
(β) when the retrotransposon or retrotransposon-like sequence is not inserted (or not present) in the modified region (particularly, when the retrotransposon or retrotransposon-like sequence is determined or estimated to be not inserted (or not present) in the modified region), determining or estimating that the genomic DNAs or the modified region-containing fragments of the genomic DNAs are not genomic DNAs of the modified cells (i) to (iii) of the present invention or cells derived therefrom or modified region-containing fragments of the genomic DNAs, or possibly not these genomic DNAs or fragments.

The present invention may involve any one of the (α) and the (β) and may involve both the (α) and the (β). Specifically, the present invention may involve: when the retrotransposon or retrotransposon-like sequence is inserted (or present) in the modified region (particularly, when the retrotransposon or retrotransposon-like sequence is determined or estimated to be inserted (or present) in the modified region), determining or estimating that the genomic DNAs or modified region-containing fragments of the genomic DNAs are genomic DNAs of the modified cells (i) to (iii) of the present invention or cells derived therefrom or modified region-containing fragments of the genomic DNAs, or possibly these genomic DNAs or fragments; and/or when the retrotransposon or retrotransposon-like sequence is not inserted (or not present) in the modified region (particularly, when the retrotransposon or retrotransposon-like sequence is determined or estimated to be not inserted (or not present) in the modified region), determining or estimating that the genomic DNAs or the modified region-containing fragments of the genomic DNAs are not genomic DNAs of the modified cells (i) to (iii) of the present invention or cells derived therefrom or modified region-containing fragments of the genomic DNAs, or possibly not these genomic DNAs or fragments. When modification at all of a plurality of sites is determined or estimated to be modification consistent with the information on modification, this means that the analyzed genomic DNAs or modified region-containing fragments of the genomic DNAs can be determined to be genomic DNAs of the modified cells (i) to (iii) of the present invention or modified region-containing fragments of the genomic DNAs with higher precision.

In an aspect, the present invention provides a method comprising:
providing readable information corresponding to each of a plurality of containers each containing a cell or genomic DNA thereof, or a modified region-containing fragment of the genomic DNA, and a correspondence table comprising information on a modified region of each cell associated with the readable information; and
identifying information on a modified region in the cell contained in the container, the genomic DNA thereof, or the modified region-containing fragment of the genomic DNA from the readable information given to the container on the basis of the correspondence table, and/or identifying a container containing a cell having the modification or genomic DNA thereof, or a modified region-containing fragment of the genomic DNA from the information on the modified region.

The genomic DNA of the cell or the modified region-containing fragment of the genomic DNA has the modification of the present invention. In this aspect, the method of the present invention may further comprise:
providing a plurality of containers each containing a cell or genomic DNA thereof, or a modified region-containing fragment of the genomic DNA, the containers being each given readable information; and
storing or preserving the containers under conditions suitable for the storage or preservation of the cell, the genomic DNA thereof, or the modified region-containing fragment of the genomic DNA.

In an aspect, the present invention also provides a method comprising:
storing (e.g., electronically storing), in a recording medium (e.g., a non-volatile memory), readable information corresponding to each of a plurality of containers each containing a cell or genomic DNA thereof, or a modified region-containing fragment of the genomic DNA, and a correspondence table comprising information on a modified region of each cell associated with the readable information; and
allowing a processor (e.g., CPU) to execute a program to identify information on a modified region in the cell contained in the container, the genomic DNA thereof, or the modified region-containing fragment of the genomic DNA from the readable information given to the container on the basis of the correspondence table, and/or to identify a container containing a cell having the modification or genomic DNA thereof, or a modified region-containing fragment of the genomic DNA from the information on the modified region in the cell.

In this aspect, the method of the present invention can be carried out using a computer. The computer can be a server connected through an electronic line such as the internet. The server may be placed in a remote location. The computer may be a computer of executing the method offline. The correspondence table can be read from a recording medium at the time of execution of the present invention.

The system may further comprise an output part that outputs information on the cell, the genomic DNA thereof, or the modified region-containing fragment of the genomic DNA, or the container containing it, identified on the basis of the correspondence table and/or the modification information on the cell, the genomic DNA thereof, or the modified region-containing fragment of the genomic DNA. Examples of the output part include a display connected to the system, a printer connected to the system, and transmission parts to other terminals (e.g., transmission parts that transmit information to other terminals via internet lines, and transmission parts that transmit information to other terminals over the wireless).

### <Animal or plant (e.g., livestock) individual or variety management system and management method>

In an aspect, the present invention can provide an animal or plant (e.g., livestock) individual or variety management system.

According to the present invention, the animal or plant (e.g., livestock) individual or variety management system can comprise:
a recording medium that stores information on each of a plurality of animal or plant (e.g., livestock) individuals or animal or plant (e.g., livestock) varieties each comprising a cell having the modification of the present invention, and a correspondence table comprising modification information on genomic DNA of each animal or plant (e.g., livestock) associated with the information;
a reception part that receives the information on the plurality of animal or plant (e.g., livestock) individuals or animal or plant (e.g., livestock) varieties, and/or the modification information on the genomic DNA; and/or
a processor capable of executing a program, wherein the program is a program to identify modification information on the genomic DNA of an animal or plant (e.g., livestock) individual or animal or plant (e.g., livestock) variety corresponding to the received information on each of the animal or plant (e.g., livestock) individuals or the animal or plant (e.g., livestock) varieties on the basis of the correspondence table read from the recording medium, and/or to identify an animal or plant (e.g., livestock) individual or animal or plant (e.g., livestock) variety having modification corresponding to the modification information on the genomic DNA.

The present invention provides a method comprising:
providing information on each of animal or plant (e.g., livestock) individuals or animal or plant (e.g., livestock) varieties, and a correspondence table comprising modification information on genomic DNA of each animal or plant (e.g., livestock) individual or animal or plant (e.g., livestock) variety associated with the information; and
identifying modification information on the genomic DNA from the information on the animal or plant (e.g., livestock) individual or the animal or plant (e.g., livestock) variety on the basis of the correspondence table, and/or identifying an animal or plant (e.g., livestock) individual or an animal or plant (e.g., livestock) variety having the modification from the modification information on the genomic DNA.

In the case of managing an animal or plant (e.g., livestock) individual and an animal or plant (e.g., livestock) variety, the animal or plant (e.g., livestock) may not be given readable information. Basically, the present invention provides a technique of identifying the origin of an animal or plant (e.g., livestock) individual or an animal or plant (e.g., livestock) variety when the animal or plant (e.g., livestock) individual or the animal or plant (e.g., livestock) variety is leaked outside the management. Information on genomic DNA suffices for the identification. Therefore, the animal or plant (e.g., livestock) individual or the animal or plant (e.g., livestock) variety itself does not have to be given readable information. In the present invention, modification information on genomic DNA can be recorded in a recording medium (non-volatile memory, etc.) so as to merely associate the information with a particular animal or plant (e.g., livestock) individual or animal or plant (e.g., livestock) variety.

The present invention can also provide a method comprising:
providing readable information corresponding to each of animal or plant (e.g., livestock) individuals or animal or plant (e.g., livestock) varieties, and a correspondence table comprising modification information on genomic DNA of each animal or plant (e.g., livestock) individual or animal or plant (e.g., livestock) variety associated with the readable information; and
identifying modification information on genomic DNA from the readable information given to the animal or plant (e.g., livestock) individual or the animal or plant (e.g., livestock) variety on the basis of the correspondence table, and/or identifying an animal or plant (e.g., livestock) individual or an animal or plant (e.g., livestock) variety having the modification from the modification information on the genomic DNA.

In the case of managing an animal or plant (e.g., livestock) individual, each animal or plant (e.g., livestock) may be given readable information, though the management is not particularly limited thereto. The readable information can be recorded in, for example, an integrated circuit for identification. The integrated circuit for identification may be attached outside the body or can be embedded in the body. The integrated circuit for identification can be, for example, a RFID apparatus (e.g., a passive RFID apparatus). The integrated circuit for identification may be coated with a biocompatible material for embedding in the body. Examples of the biocompatible material include, but are not particularly limited to, soda-lime glass and borosilicate glass.

The system may further comprise an output part that outputs information on the animal or plant (e.g., livestock) individual or the animal or plant (e.g., livestock) variety identified on the basis of the correspondence table and/or the modification information on the genomic DNA of the animal or plant (e.g., livestock) individual or the animal or plant (e.g., livestock) variety. Examples of the output part include a display connected to the system, a printer connected to the system, and transmission parts to other terminals (e.g., transmission parts that transmit information to other terminals via internet lines, and transmission parts that transmit information to other terminals over the wireless).

On the basis of information on modification, sequence decoding can be performed by a method well known to those skilled in the art. For example, the positional relationship of a modification site with a neighboring sequence can be determined on the basis of information on the neighboring sequence. The determination can be performed by sequencing. In a preferred aspect, the determination can be performed by amplifying a gene including the neighboring sequence by a gene amplification method (e.g., polymerase chain reaction (PCR)), and then sequencing the amplification product. The gene amplification can be performed using, for example, (a) primers for neighboring sequences outside the retrotransposon or retrotransposon-like sequence, and the sequence of the retrotransposon or retrotransposon-like sequence flanked by the neighboring sequences can be amplified by the amplification. In this way, the insertion position of the retrotransposon or retrotransposon-like sequence and/or the sequence of the retrotransposon or retrotransposon-like sequence can be determined by determining the sequence of a boundary site between the retrotransposon or retrotransposon-like sequence and its neighboring region. Alternatively, the gene amplification can be performed using, for example, (b) a primer for the inside of the retrotransposon or retrotransposon-like sequence and a primer for an outside neighboring sequence, and a sequence including a boundary site between the neighboring sequence and the retrotransposon or retrotransposon-like sequence can be amplified by the amplification. In this way, the insertion position of the retrotransposon or retrotransposon-like sequence can be determined by determining the sequence of the boundary site between the retrotransposon or retrotransposon-like sequence and the neighboring sequence. In a preferred aspect, the determination can be performed by cleaving genomic DNA with a restriction enzyme, then preparing circular DNA by ligation, and subjecting the circular DNA to inverse PCR from the inside toward the outside of the inserted sequence which is the retrotransposon or retrotransposon-like sequence to obtain an amplification product, and determining the sequence of the amplification product.

Thus, the present invention provides
a method for analyzing the origin of a cell, the method comprising:
decoding a nucleotide sequence of a target region in genomic DNA of the test cell suspected of being derived from the modified cell of the present invention, or providing the decoded nucleotide sequence;
determining the presence or absence of a nucleotide sequence introduced in the modified region on the basis of the decoded nucleotide sequence; and
when the nucleotide sequence introduced in the modified region is determined or estimated to be present, determining or estimating that the test cell is derived or possibly derived from the modified cell of the present invention, and/or in other cases, determining or estimating that the test cell is not derived or possibly not derived from the modified cell of the present invention.

The present invention also provides
a method for analyzing the origin of a cell, the method comprising:
decoding a nucleotide sequence of a target region in genomic DNA of the test cell suspected of being derived from the modified cell of the present invention, or providing the decoded nucleotide sequence;
determining the presence or absence of a nucleotide sequence introduced in the modified region on the basis of the decoded nucleotide sequence; and
when the nucleotide sequence introduced in the modified region is determined or estimated to be present, determining or estimating that the test cell is derived or possibly derived from the modified cell of the present invention, and/or in other cases, determining or estimating that the test cell is not derived or possibly not derived from the modified cell of the present invention.

The present invention further provides
a method for analyzing the origin of a cell, the method comprising:
decoding a nucleotide sequence of a target region in genomic DNA of the test cell suspected of being derived from the modified cell of the present invention (hereinafter, also referred to as the "cell of origin"), or providing the decoded nucleotide sequence;
optionally determining whether or not a modified nucleotide sequence is inserted in the modified region on the basis of the decoded nucleotide sequence; and
when the nucleotide sequence introduced in the modified region is determined or estimated to be present, determining or estimating that the test cell is derived or possibly derived from the modified cell of the present invention, and/or in other cases, determining or estimating that the test cell is not derived or possibly not derived from the modified cell of the present invention.

The nucleotide sequence of the target sequence in the genomic DNA can be decoded on the basis of the modified region. Specifically, the modification site contained in the modified region can be selectively amplified or isolated and sequenced on the basis of information on the modification site.

Likewise, the present invention provides a method for analyzing the origin of genomic DNA having the modification of the present invention or a modified region-containing fragment of the genomic DNA. The present invention provides
a method for analyzing the origin of genomic DNA or a modified region-containing fragment of the genomic DNA, the method comprising:
decoding a nucleotide sequence of a target region in genomic DNA of a cell suspected of being derived from the modified cell of the present invention or a modified region-containing fragment of the genomic DNA, or providing the decoded nucleotide sequence;
determining the presence or absence of a nucleotide sequence introduced in the modified region on the basis of the decoded nucleotide sequence; and
when the nucleotide sequence introduced in the modified region is determined or estimated to be present, determining or estimating that the genomic DNA or the modified region-containing fragment of the genomic DNA is or possibly is genomic DNA of a cell derived from the modified cell of the present invention or a modified region-containing fragment of the genomic DNA, and/or in other cases, determining or estimating that the genomic DNA or the modified region-containing fragment of the genomic DNA is or possibly is genomic DNA of a cell that is not derived from the modified cell of the present invention or a modified region-containing fragment of the genomic DNA.

The present invention also provides
a method for analyzing the origin of genomic DNA or a modified region-containing fragment of the genomic DNA, the method comprising:
decoding a nucleotide sequence of a target region in genomic DNA of a cell suspected of being derived from the modified cell of the present invention or a modified region-containing fragment of the genomic DNA, or providing the decoded nucleotide sequence;
determining the presence or absence of a nucleotide sequence introduced in the modified region on the basis of the decoded nucleotide sequence; and
when the nucleotide sequence introduced in the modified region is determined or estimated to be present, determining or estimating that the genomic DNA or the modified region-containing fragment of the genomic DNA is or possibly is genomic DNA of a cell derived from the modified cell of the present invention or a modified region-containing fragment of the genomic DNA, and/or in other cases, determining or estimating that the genomic DNA or the modified region-containing fragment of the genomic DNA is or possibly is genomic DNA of a cell that is not derived from the modified cell of the present invention or a modified region-containing fragment of the genomic DNA.

The present invention further provides
a method for analyzing the origin of genomic DNA or a modified region-containing fragment of the genomic DNA, the method comprising:
decoding a nucleotide sequence of a target region in genomic DNA of a cell suspected of being derived from the modified cell of the present invention (hereinafter, also referred to as the "cell of origin") or a modified region-containing fragment of the genomic DNA, or providing the decoded nucleotide sequence;
optionally determining whether or not a modified nucleotide sequence is inserted in the modified region on the basis of the decoded nucleotide sequence; and
when the nucleotide sequence introduced in the modified region is determined or estimated to be present, determining or estimating that the genomic DNA or the modified region-containing fragment of the genomic DNA is or possibly is genomic DNA of a cell derived from the modified cell of the present invention or a modified region-containing fragment of the genomic DNA, and/or in other cases, determining or estimating that the genomic DNA or the modified region-containing fragment of the genomic DNA is or possibly is genomic DNA of a cell that is not derived from the modified cell of the present invention or a modified region-containing fragment of the genomic DNA.

The nucleotide sequence of the target sequence in the genomic DNA can be decoded on the basis of the modified region. Specifically, the modification site contained in the modified region can be selectively amplified or isolated and sequenced on the basis of information on the modification site.

In an aspect, the presence or absence of a nucleotide sequence introduced in the modified region can be evaluated on the basis of information on the modified region (e.g., modification information). Whether or not an introduced nucleotide sequence is inserted in the modified region can be determined from, for example, the presence or absence of an insert at a position supposed to have the insert (or a designed position). Retrotransposons continue to spontaneously increase their own copies in genomic DNA. Thus, mere presence of a retrotransposon sequence at a new position does not determine artificial modification. However, a sequence, when inserted in the modified region (or at a designed position or an intended position) without shift even by one base, is likely to be an artificial insert. For example, human genomic DNA has a length of 3 billion base pairs, and, for example, the probability of insertion of a retrotransposon at a correct position without shift even by one base is one three-billionth. Therefore, when a retrotransposon is inserted at a correct position without shift even by one base, the possibility can be stochastically eliminated that the insertion is accidental insertion resulting from natural replication. Thus, in an aspect, whether or not a sequence is inserted in the modified region can be confirmed from, for example, insertion at a designed position or an intended position without shift (insertion or deletion) even by one base. However, in actuality, the insertion, deletion, or substitution of a nucleotide sequence on the order of one base to several bases can occur at a low frequency during culture. Thus, in analysis, the sequence may be estimated to be inserted at a designed position or intended position even if there exists shift or difference of approximately one base to several bases (e.g., 1 to 5 bases, preferably 1 to 2 bases, more preferably 1 base). Thus, in an aspect, confirmation on whether an introduced nucleotide sequence is inserted in the modified region further involves estimating or determining that the sequence is inserted at a designed position or an intended position when shift or substitution from the designed position is approximately one base to several bases (preferably 1 to 2 bases, more preferably 1 base). This is because the probability of spontaneous and accidental insertion of a retrotransposon or retrotransposon-like sequence to a location that differs by several bases is almost negligibly low in consideration of a genome size. If a second retrotransposon or retrotransposon-like sequence is further introduced to the genomic DNA, and the insertion of the sequence at a correct position is further confirmed, and then all of a plurality of inserts are present at correct positions, the possibility of spontaneous occurrence of these inserts can be strongly eliminated. The number of modification can be, for example, 2 or more, can be, for example, 3 or more, or can be, for example, 4 or more. Basically, a retrotransposon does not disappear from its insertion site because of the nature thereof, and the insertion can be permanently maintained. Although the sequence might be further mutated by subsequent culture or the like, the introduction position is basically kept constant as described above. Thus, in an aspect of the present invention, whether or not a cell is derived from the modified cell can be determined or estimated on the basis of the introduction position. In the case of determining or estimating whether or not a cell is derived from the modified cell on the basis of information on the introduced nucleotide sequence, the examined cell can be determined or estimated to be derived from the modified cell even if there exists difference by one to several bases. Smaller difference enhances specificity while larger difference enhances sensitivity. Thus, trade-off occurs between specificity and sensitivity. Those skilled in the art can determine an acceptable magnitude of the difference such that preferred specificity and sensitivity can be obtained according to a purpose. In an aspect, the genome in which the retrotransposon or retrotransposon-like sequence is inserted in a site-specific manner by inserting donor DNA to a DNA cleavage site may be confirmed to be the genome having an insert of the donor DNA, or not, on the basis of whether or not one of the strands (single strand) of the donor DNA can be hybridized.

As described above, the leak of an important cell resource outside the management can be detected, and the leaked cell can be identified. It is considered that the genomic DNA of the cell is not changed by subsequent culture, particularly, proliferation and differentiation (or dedifferentiation). Thus, modified cell-derived cells such as proliferated cells and differentiated (or dedifferentiated) cells can also be estimated to have the same genomic DNA as that of the modified cell. Thus, according to the present invention, a cell can be determined to be derived or not derived from an important cell resource leaked outside the management. This method can be effective for performing cell distribution management (e.g., management to prohibit use of cells for a purpose other than the licensed one, and management to prohibit cell loan or assignment to a person other than the licensed one). For example, when cells are leaked outside the management, the method can be used for analyzing where the cells have been leaked from and identifying the outflow source, by obtaining and analyzing the cells. For example, when certain cells are loaned or assigned to a plurality of recipients, cells having different modification among the recipients can be loaned or assigned. A correspondence table showing the correspondence relationship between modification information and each recipient can be prepared and recorded or stored in a recording medium. If cells are leaked outside the management, the genomic DNA of the cells suspected of having been leaked is analyzed on the basis of the modification information and whether or not the modification is based on any modification information described in the correspondence table is examined. When modification information corresponding to the modification of the genomic DNA is discovered, the leaked cells can be determined or estimated to be from the recipient of the cells having the modification corresponding to the modification information. Such cell distribution management can be carried out by, for example, a cell assigner or loaner.

In a preferred aspect, the cell can be sperm. For example, sperm (e.g., livestock sperm, for example, bovine (e.g., Japanese cattle) sperm or swine sperm), for example, sex-sorted semen, can be analyzed, stored or preserved, and/or managed by the method of the present invention. Sperm having modified genomic DNA can be obtained from a cell or an individual having the modified genomic DNA by the method of the present invention.

### <Eukaryote comprising at least any one of modified cells (i) to (iii) of present invention>

The present invention provides a eukaryote having at least any one of the modified cells (i) to (iii) of the present invention or genomic DNA thereof. The eukaryote is not particularly limited and can be an animal or a plant. Examples of the animal include agricultural animals such as mammals (non-human mammals), particularly, livestock, for example, bovines (e.g., Japanese cattle), pigs, goats, sheep, horses, llamas, and camels, birds such as chickens, and seafood such as fish, pet animals such as dogs, cats, rabbits, guinea pigs, hamsters, and mice, and insects. Examples of the plant include agricultural crops, for example, edible crops, for example, rice, corn, tubers and roots, beans, barley, and wheat, horticultural crops (vegetables, fruit trees, and flowers), for example, leaf vegetables (cabbage, asparagus, etc.), fruit vegetables (eggplant, tomato, cucumbers, etc.), root vegetables (radish, carrot, etc.), other vegetables, fruits (e.g., pomaceous fruits such as apple and pear, stone fruits such as Japanese apricot, apricot, prune, peach, and cherry, nuts such as almond, walnut, and Japanese chestnut, citruses such as tangerine and lemon, strawberries such as *Fragaria L.,* and tropical fruit trees such as tropical fruits), and ornamental plants, and seeds, seedlings, and bulbs of any of these plants. The eukaryote can be a non-human animal. In this way, whether or not a eukaryote has at least any one of the modified cells (i) to (iii) of the present invention or genomic DNA thereof can be determined. The eukaryote can be obtained in accordance with a usual method for preparing a recombinant animal (e.g., somatic cell nuclear transfer or pluripotent cell editing).

This enables a eukaryote to be managed or tracked. When such tracking is possible, the leak of a resource such as an important agricultural crop or livestock outside the management can be detected, and the leaked resource can be identified. The method of the present invention can also be applied to animal or plant (e.g., livestock) varieties, for example, agricultural crop or livestock (e.g., Japanese cattle or strawberry) varieties. As one example, a registration organization or a certification organization, for example, an international registration organization or certification organization, is established in which the modification information on genomic DNA according to the method of the present invention is registered. A preparer of at least any one of the modified cells (i) to (iii) of the present invention can register the modification information on genomic DNA according to the method of the present invention in the registration organization or the certification organization. A cell prepared together therewith or genomic DNA thereof may be deposited in the registration organization or the certification organization. In the registration organization or the certification organization, for example, the modification information on genomic DNA is kept confidential and managed. When someone sends a cell and modification information to the registration organization or the certification organization and requests analysis thereon, the registration organization or the certification organization can determine whether or not a eukaryote to be examined or its cell has or is a cell derived from the cell prepared on the basis of the modification information sent by the client, and send back the results to the client.

## Claims

1. A method for obtaining a cell, wherein
the cell comprises genomic DNA, and the genomic DNA comprises nucleotide sequences of a plurality of retrotransposons,
the method comprising:
(i) newly introducing a nucleotide sequence having 50% or more sequence identity to any one of the nucleotide sequences of the retrotransposons in the genomic DNA (i.e., a retrotransposon or retrotransposon-like nucleotide sequence) into a target sequence in the genomic DNA, and selecting a cell comprising the genomic DNA with the retrotransposon or retrotransposon-like sequence integrated in the target sequence;
(ii) changing an insertion position, to each neighboring sequence, of at least one of the nucleotide sequences of the retrotransposons in the genomic DNA, and selecting a cell comprising the genomic DNA having the nucleotide sequence of the retrotransposon with the insertion position changed; and/or
(iii) modifying (nucleotide sequence modification selected from insertion, substitution, deletion, addition, and elimination) nucleotide sequences of one or more transposons of the genomic DNA, and selecting a cell in which the nucleotide sequence of the transposon at a particular position of the genomic DNA is a sequence different from the original one.

2. The method according to claim 1, wherein in the (i), the new introduction into the target sequence comprises introducing the retrotransposon or retrotransposon-like sequence into the target sequence of the genomic DNA through the use of nucleotide sequence-specific cleavage and subsequent homologous recombination, and selecting a cell comprising the genomic DNA with the retrotransposon or retrotransposon-like sequence integrated in the target sequence.

3. The method according to claim 2, wherein in the (i), the new introduction into the target sequence further comprises, upon the introduction, removing a portion or the whole of an artificial sequence integrated in the genomic DNA from the genomic DNA, and selecting a cell from which a portion or the whole of the integrated artificial sequence has been removed.

4. The method according to any one of claims 1 to 3, wherein the nucleotide sequence to be newly introduced in the (i) has 90% or more sequence identity to any one of the nucleotide sequences of the retrotransposons in the genomic DNA.

5. The method according to any one of claims 1 to 4, wherein the nucleotide sequence to be newly introduced in the (i) is a nucleotide sequence identical to any one of the nucleotide sequences of the retrotransposons in the genomic DNA.

6. The method according to any one of claims 1 to 5, further comprising (i) introducing a nucleotide sequence (second nucleotide sequence) having 50% or more sequence identity to any one of the nucleotide sequences of the retrotransposons in the genomic DNA into another target sequence (second target sequence) in the genomic DNA.

7. A cell having, in a target sequence in genomic DNA, an additional nucleotide sequence having 50% or more sequence identity to any one of nucleotide sequences of retrotransposons in the genomic DNA.

8. The cell according to claim 7, wherein the additional nucleotide sequence is a non-natural nucleotide sequence.

9. A method for analyzing the origin of a cell or genomic DNA thereof, or a modified region-containing fragment of the genomic DNA, the method comprising:
decoding a nucleotide sequence of a modified region in the genomic DNA of the test cell suspected of being derived from the cell according to claim 7 or 8 (cell of origin), the test genomic DNA, or the modified region-containing fragment of the genomic DNA, or providing information on the decoded nucleotide sequence;
determining or estimating the presence or absence of a nucleotide sequence introduced in the modified region on the basis of the decoded nucleotide sequence and information on the modified region; and
when the nucleotide sequence introduced in the modified region is determined or estimated to be present, determining or estimating that the test cell is derived or possibly derived from the cell according to claim 7 or 8 (the cell of origin), and/or in other cases, determining or estimating that the test cell is not derived or possibly not derived from the cell according to claim 7 or 8 (the cell of origin).

10. A non-human organism comprising the cell according to claim 7 or 8.

11. A cell management system comprising a plurality of containers each containing the cell according to claim 7 or 8, wherein the containers have readable information corresponding to each of the containers, and further have a correspondence table comprising a modified region of each cell associated with the readable information.

12. A method comprising:
providing readable information corresponding to each of a plurality of containers each containing the cell according to claim 7 or 8 or genomic DNA thereof, or a modified region-containing fragment of the genomic DNA, and a correspondence table comprising information on a modified region of each cell associated with the readable information; and
identifying information on a modified region in the cell contained in the container, the genomic DNA thereof, or the modified region-containing fragment of the genomic DNA from the readable information given to the container on the basis of the correspondence table, and/or identifying a cell having the modification or genomic DNA thereof, or a modified region-containing fragment of the genomic DNA, or a container containing it from the information on the modified region.

13. The method according to claim 12, further comprising:
providing a plurality of containers each containing the cell according to claim 7 or 8 or genomic DNA thereof, or a modified region-containing fragment of the genomic DNA, the containers being each given readable information; and
storing or preserving the containers under conditions suitable for the storage or preservation of the cell, the genomic DNA thereof, or the modified region-containing fragment of the genomic DNA.

14. A method comprising:
providing information on each of animal or plant individuals or varieties each comprising the cell according to claim 7 or 8, and a correspondence table comprising modification information on genomic DNA of each animal or plant individual or variety associated with the information; and
identifying modification information on the genomic DNA from the information on the animal or plant individual or variety on the basis of the correspondence table, and/or identifying an animal or plant individual or variety having the modification from the modification information on the genomic DNA.

15. A management system of information on a cell or genomic DNA thereof, or a modified region-containing fragment of the genomic DNA, the management system comprising:
a recording medium that stores readable information corresponding to each of a plurality of containers each containing the cell according to claim 7 or 8 or genomic DNA thereof, or a modified region-containing fragment of the genomic DNA, and a correspondence table comprising modification information on each cell associated with the readable information;
a reception part that receives the readable information and/or the modification information; and
a processor capable of executing a program, wherein the program is a program to identify modification information on the cell contained in the container having the received readable information, the genomic DNA thereof, or the modified region-containing fragment of the genomic DNA on the basis of the correspondence table read from the recording medium, and/or to identify a cell or genomic DNA thereof, or a modified region-containing fragment of the genomic DNA, or a container containing it from the modification information.

16. An animal or plant individual or variety management system comprising:
a recording medium that stores information on each of a plurality of animal or plant individuals or varieties each comprising the cell according to claim 7 or 8, and a correspondence table comprising modification information on genomic DNA of each animal or plant individual or variety associated with the information;
a reception part that receives the information on each of the plurality of animal or plant individuals or varieties, and/or the modification information on the genomic DNA; and/or
a processor capable of executing a program, wherein the program is a program to identify modification information on the genomic DNA of an animal or plant (e.g., livestock) individual or variety corresponding to the received information on each of the animal or plant individuals or varieties on the basis of the correspondence table read from the recording medium, and/or to identify an animal or plant individual or variety having modification corresponding to the modification information on the genomic DNA.
